(19)

Europäisches
Patentamt
European
Patent Office
Office européen
des brevets

(11) **EP 4 316 655 A1**

(12) **EUROPEAN PATENT APPLICATION**
published in accordance with Art. 153(4) EPC

(43) Date of publication:
**07.02.2024 Bulletin 2024/06**

(21) Application number: **22781043.9**

(22) Date of filing: **30.03.2022**

(51) International Patent Classification (IPC):
$B01J\ 23/755^{(2006.01)}$    $B01J\ 35/10^{(2006.01)}$
$B01J\ 37/08^{(2006.01)}$    $C01B\ 3/02^{(2006.01)}$
$C01B\ 32/40^{(2017.01)}$    $C07B\ 61/00^{(2006.01)}$
$C07C\ 1/04^{(2006.01)}$    $C07C\ 9/04^{(2006.01)}$
$C07C\ 9/06^{(2006.01)}$    $C07C\ 9/08^{(2006.01)}$
$C07C\ 9/10^{(2006.01)}$    $C25B\ 1/042^{(2021.01)}$
$C25B\ 1/23^{(2021.01)}$    $C25B\ 9/23^{(2021.01)}$
$B01J\ 23/46^{(2006.01)}$    $B01J\ 23/648^{(2006.01)}$
$B01J\ 23/652^{(2006.01)}$    $C25B\ 11/052^{(2021.01)}$
$C25B\ 11/054^{(2021.01)}$    $C25B\ 11/067^{(2021.01)}$
$C25B\ 11/075^{(2021.01)}$

(52) Cooperative Patent Classification (CPC):
**B01J 23/755; B01J 35/60; B01J 37/08; C01B 3/02;
C01B 32/40; C07B 61/00; C07C 1/04; C07C 9/04;
C07C 9/06; C07C 9/08; C07C 9/10; C25B 1/042;
C25B 1/23; C25B 9/23;** B01J 23/46;    (Cont.)

(86) International application number:
**PCT/JP2022/015817**

(87) International publication number:
**WO 2022/210832 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2021 JP 2021062170**

(71) Applicant: **Osaka Gas Co., Ltd.
Osaka-shi, Osaka 541-0046 (JP)**

(72) Inventors:
- **ECHIGO Mitsuaki
  Osaka-shi, Osaka 541-0046 (JP)**
- **TSUDA Yuji
  Osaka-shi, Osaka 541-0046 (JP)**
- **HIRANO Takenori
  Izumi-shi, Kagoshima 899-0401 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **METHOD FOR MANUFACTURING REVERSE WATER-GAS SHIFT CATALYST, REVERSE WATER-GAS SHIFT CATALYST, ELECTROLYTIC REACTION SYSTEM, HYDROCARBON MANUFACTURING SYSTEM, AND METHOD FOR USING REVERSE WATER-GAS SHIFT CATALYST**

(57) A reverse water-gas shift catalyst that can be used at high temperatures is obtained. A reverse water-gas shift catalyst (cat1) is produced by executing an impregnation-supporting step of impregnating a carrier (cb1) containing alumina as a main component with nickel as a catalytically active component (ca1) to be supported on the carrier, and calcinating a precursor obtained in the impregnation-supporting step at a temperature of 500°C or higher.

Fig.11

(a) IMPREGNATION-SUPPORTING STEP — cb1,cb2 / ca1,ca2

(b) DRYING/CRUSHING/MOLDING STEP — ca1,ca2 / cb1,cb2

(c) CALCINATION STEP — ca1,ca2 / cb1,cb2

cat1,cat2

(52) Cooperative Patent Classification (CPC): (Cont.)
B01J 23/648; B01J 23/652; C25B 11/052;
C25B 11/054; C25B 11/067; C25B 11/075

**Description**

Technical Field

The present invention relates to a reverse water-gas shift catalyst and a method for producing the same, and also relates to an electrolysis reaction system and a hydrocarbon production system including a catalytic reaction unit using this kind of reverse water-gas shift catalyst.

Background Art

In the production of hydrocarbons, a technique is known in which carbon monoxide and hydrogen are used as raw material gases and methane gas is obtained from these gases.

PTL 1 discloses a system for producing the carbon monoxide which is one of the raw material gases.

The system disclosed in PTL 1 includes an electrolysis device (corresponding to an electrolysis reaction unit of the present invention) that performs electrolysis treatment, supplies carbon dioxide and water to a cathode of this device, and generates hydrogen and carbon monoxide by electrolysis. In this reaction, unreacted water and carbon dioxide remain. Therefore, hydrogen, carbon monoxide, unreacted water, and carbon dioxide sent from the electrolysis device are sent to a reverse shift reactor (corresponding to a reverse water-gas shift reaction unit of the present invention) to react carbon dioxide with hydrogen and generate carbon monoxide and water.

The electrolysis device operates at about 700 to 900°C, the reverse shift reactor operates at about 600 to 950°C, and copper (Cu), nickel (Ni), or the like can be used as the catalyst (paragraph [0029] of the specification).

Citation List

Patent Literature

[PTL 1] JP-A-2019-35102

Summary of Invention

Technical Problem

The catalyst used here is also called a reverse shift catalyst, and is a catalyst which performs a reverse reaction (reaction that generates carbon monoxide and water from carbon dioxide and hydrogen) of the following water-gas shift reaction (reaction that generates carbon dioxide and hydrogen from carbon monoxide and water), that is, a reverse water-gas shift reaction.

$$CO + H_2O \rightarrow CO_2 + H_2 \ \Delta H = -41 \ kJ/mol$$

The former water-gas shift reaction is a reaction widely used in hydrogen production processes and the like, and is a reaction in which carbon monoxide is reacted with water to convert into carbon dioxide and hydrogen. However, since the reaction is an exothermic reaction, equilibrium shifts to a carbon dioxide generation side as the reaction is performed at a lower temperature. Therefore, in the related art, a catalyst with enhanced low-temperature activity has been developed. Generally, iron-chromium catalysts used at about 300 to 450°C and copper-zinc catalysts used at about 200°C are known.

Meanwhile, since there are not many applications that require the reverse reaction of the water-gas shift reaction (reverse water-gas shift reaction), development of a catalyst suitable for the reverse water-gas shift reaction has not been carried out so far.

The reverse water-gas shift reaction is a reaction in which carbon dioxide is reacted with hydrogen to convert into carbon monoxide and water. However, this reaction is an endothermic reaction, and in order to move the equilibrium to the carbon monoxide generation side, unlike the water-gas shift reaction, a high temperature is required, and thus, there is a need for a catalyst that can be used at as high a temperature as possible. However, from this point of view, development of high-performance catalysts suitable for the reverse water-gas shift reaction has not been carried out much.

An object of the present invention is to obtain a reverse water-gas shift catalyst that can be used at a high temperature and to obtain a method for producing the revers water gas shift catalyst. In addition, it is to provide a system capable of obtaining raw materials for use in, for example, hydrocarbon production, using a reverse water-gas shift reaction together with an electrolysis reaction.

Solution to Problem

According to a first characteristic configuration of the present invention, there is provided a method for producing a reverse water-gas shift catalyst, the method comprising calcinating a precursor obtained by adding at least a nickel component to alumina at a temperature of 500°C or higher.

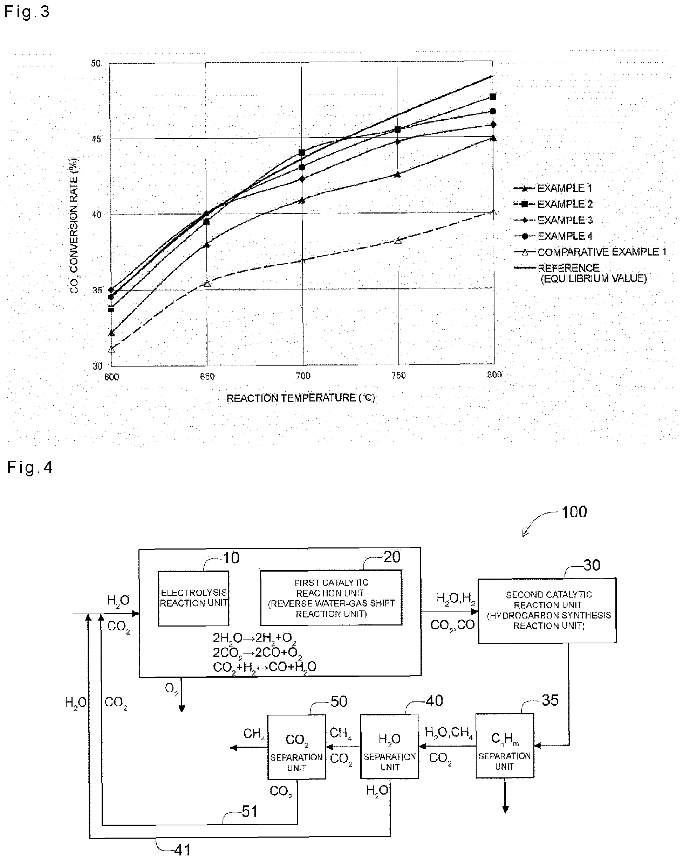

As will be described later, various catalysts were investigated as candidates for the reverse water-gas shift catalyst, and as such candidates, a catalyst containing nickel and aluminum obtained through a calcination process was also investigated. In this investigation, the calcination temperature was increased from 450°C to 1000°C. As a result, the inventors have completed the present invention based on the discovery that while activity of the catalyst calcinated at 450°C was low, when the calcination temperature was raised above 500°C, the catalytic activity increased step by step and a $CO_2$ conversion rate reached the vicinity of an equilibrium value (calculated value). Fig. 3 illustrates results of a sample with a calcination temperature of 450°C and results of samples with a calcination temperature increased to 600°C, 800°C, 1000°C, and 1200°C. For reference, Fig. 3 also illustrates the equilibrium value (calculated value) of the $CO_2$ conversion rate.

The catalyst was prepared through a step of calcinating a precursor obtained through a so-called impregnation-supporting step. However, from the results illustrated in Fig. 3, since there is a clear difference in properties between the sample calcinated at 450°C and the samples calcinated at 500°C or higher (600°C, 800°C, 1000°C, and 1200°C), it is considered that there will be a clear difference in the structures of the catalyst between the two. That is, in the sample calcinated at 450°C, while nickel was simply distributed on the surface of the alumina, when calcinated at 500°C or higher, a composite oxide (a composite oxide with a spinel structure) of nickel and alumina was generated, and it is speculated that the activity is enhanced step by step. Since this catalyst becomes a highly active catalyst on the relatively high temperature side and the calcinating operation is performed on the high temperature side, even when the catalyst is used in a high temperature range (for example, 600°C to 800°C) where the above equilibrium reaction proceeds toward the reverse water-gas shift reaction, problems such as progress of sintering of the catalyst are unlikely to occur.

Furthermore, this catalyst was compared with an example using platinum, which is an expensive noble metal, the catalyst according to the present invention showed comparable activity.

The inventors have recently confirmed the above facts for the first time and completed the present invention.

Therefore, when producing a reverse water-gas shift catalyst, as described as a second characteristic configuration of the present invention, an impregnation-supporting step of impregnating a carrier containing alumina as a main component with nickel to be supported on the carrier is executed, and a precursor obtained in the impregnation-supporting step can be calcinated at a temperature of 500°C or higher.

Moreover, the reverse water-gas shift catalyst thus obtained had the following characteristics.

(1) The catalyst contains a $NiAl_2O_4$ phase as a crystalline phase (third characteristic configuration of the present invention and seventh characteristic configuration of the present invention).

(2) A CO adsorption amount per 1 g of the catalyst is 0.45 mL or less (fourth characteristic configuration of the present invention).

(3) Nickel has an average crystal particle size of 10 nm or more (fifth characteristic configuration of the present invention).

(4) A BET surface area is 105 $m^2$ or less (sixth characteristic configuration of the present invention).

In an eighth characteristic configuration of the present invention,
there is provided an electrolysis reaction system including at least: a reverse water-gas shift reaction unit including at least the reverse water-gas shift catalyst as described above; and an electrolysis reaction unit.

According to this characteristic configuration, at least water is electrolyzed by the electrolysis reaction unit, and using the generated hydrogen, carbon dioxide can be converted into carbon monoxide by the reverse water-gas shift catalyst according to the present invention, and a raw material (at least hydrogen and carbon monoxide) used in hydrocarbon synthesis can be obtained.

In this configuration, by adopting the reverse water-gas shift catalyst according to the present invention, which can obtain sufficient activity on a high temperature side, for the reverse water-gas shift reaction unit, for example, hydrocarbons can be efficiently produced by generating hydrogen and carbon monoxide necessary for hydrocarbon synthesis. As will be described later, it is particularly preferable that a ratio of hydrogen and carbon monoxide can be adjusted by a reverse water-gas shift reaction.

Further, the heat (energy) of the electrolysis reaction unit can be effectively used in the reverse water-gas shift reaction unit.

Therefore, as described in an eleventh characteristic configuration of the present invention, when the hydrocarbon synthesis reaction unit is provided in addition to the electrolysis reaction unit and the reverse water-gas shift reaction unit, it is possible to construct a hydrocarbon production system that synthesizes a hydrocarbon using the generated hydrogen and carbon monoxide.

In a ninth characteristic configuration of the present invention,
the electrolysis reaction unit has an electrolysis cell unit in which at least an electrode layer, an electrolyte layer, and a counter electrode layer are formed on a support.

According to this characteristic configuration, as the electrolysis cell used in the electrolysis reaction unit, for example, the thin-film electrode layer, electrolyte layer, and counter electrode layer are provided on a robust support having sufficient strength even if the support is thin. Therefore, the electrolysis reaction can be effectively caused while reducing the amount of the material used to form these layers to be the electrolysis cell. As a result, it is possible to configure an electrolysis cell unit that is compact, has high performance, and has excellent strength and reliability. Metals and ceramics can be selected as constituent materials of this type of support.

In a tenth characteristic configuration of the present invention, the support is a metal.

By adopting a metal as the support, a material cost is suppressed by securing the strength with an inexpensive metal material, and it is easier to process than ceramics.

In a twelfth characteristic configuration of the present invention,
the reverse water-gas shift catalyst described so far is subjected to a reaction after performing a reduction pretreatment.

Even though the reverse water-gas shift catalyst according to the present invention is produced through a calcination treatment and in many cases, at least a part of this catalyst is in an oxide state, in this state, before the reverse water-gas shift reaction occurs (before use), by exposing the catalyst to a reducing gas such as hydrogen and performing reduction pretreatment on the catalyst, a catalytically active component in the oxidized state is reduced, and the catalytic activity can be exhibited satisfactorily.

Brief Description of Drawings

Fig. 1 is a diagram illustrating the configuration of a hydrocarbon production system.
Fig. 2 is a schematic diagram illustrating a configuration of an electrolysis reaction unit.
Fig. 3 is a diagram illustrating a $CO_2$ conversion rate at different calcination temperatures.
Fig. 4 is a diagram illustrating the configuration of a system in which the electrolysis reaction unit and a reverse water-gas shift reaction unit are integrated.
Fig. 5 is a schematic diagram of an electrolysis cell unit including the electrolysis reaction unit and the reverse water-gas shift reaction unit.
Fig. 6 is a configuration diagram of a system equipped with a heat exchanger between the electrolysis reaction unit and the reverse water-gas shift reaction unit.
Fig. 7 is a diagram illustrating another configuration of the hydrocarbon production system that guides $CO_2$ to the reverse water-gas shift reaction unit.
Fig. 8 is a diagram illustrating another configuration of the hydrocarbon production system equipped with a hydrogen separation unit.
Fig. 9 is a diagram illustrating still another configuration of the hydrocarbon production system equipped with a water separation unit in front of a hydrocarbon synthesis reaction unit.
Fig. 10 is a diagram illustrating still another configuration of the hydrocarbon production system in which only water is introduced into the electrolysis reaction unit.
Fig. 11 is an explanatory diagram illustrating a preparation state of a catalyst.
Fig. 12 is an explanatory diagram illustrating a coating/calcinated state and reduction pretreatment of a catalyst.
Fig. 13 is a schematic diagram of an electrolysis cell unit including the electrolysis reaction unit, the reverse water-gas shift reaction unit, and the hydrocarbon synthesis reaction unit.
Fig. 14 is a diagram illustrating X-ray diffraction results of a nickel/alumina catalyst before use.
Fig. 15 is a diagram illustrating X-ray diffraction results of the nickel/alumina catalyst after use.
Fig. 16 is a diagram illustrating the relationship of a $CO_2$ conversion rate with increasing an operation time.

Description of Embodiments.

An embodiment of the present invention will be described with reference to the drawings.

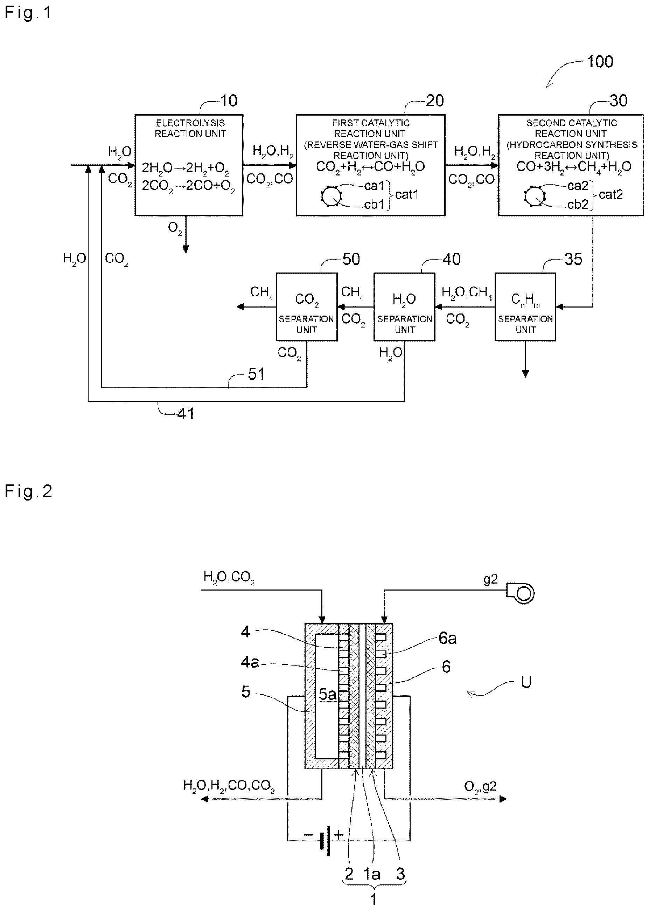

Fig. 1 illustrates a configuration of one form of a hydrocarbon production system 100 proposed by the inventors.

As illustrated in Fig. 1, the hydrocarbon production system 100 includes an electrolysis reaction unit 10, a first catalytic reaction unit 20, a second catalytic reaction unit 30, a heavy hydrocarbon separation unit 35 (illustrated as a CnHm separation unit), a water separation unit 40 (illustrated as an $H_2O$ separation unit), and a carbon dioxide separation unit 50 (illustrated as a $CO_2$ separation unit) in this order.

The electrolysis reaction unit 10 is a unit that electrolyzes at least a portion of an inflowing gas, the first catalytic reaction unit 20 is a reverse water-gas shift reaction unit that carries out a reverse water-gas shift reaction of at least a portion of the inflowing gas, and the second catalytic reaction unit 30 is configured to act as a hydrocarbon synthesis reaction unit that synthesizes at least a portion of the inflowing gas into hydrocarbon. Here, the hydrocarbon synthesized is mainly $CH_4$ (hydrocarbon having one carbon atom), but also includes other lower saturated hydrocarbons having two to four carbon atoms and the like. Further, as will be illustrated later, by appropriately selecting a catalyst used for the second catalytic reaction unit 30, heavy hydrocarbons having a larger number of carbon atoms than the lower saturated hydrocarbons, unsaturated hydrocarbons, oxygen-containing hydrocarbons, or the like can also be synthesized. Therefore, in the present specification, the hydrocarbon is a concept including all of them, and is also collectively referred to as hydrocarbons.

The heavy hydrocarbon separation unit 35, the water separation unit 40, and the carbon dioxide separation unit 50 are units for removing at least a portion of predetermined components (CnHm, $H_2O$, and $CO_2$ in the order of description) from the gas flowing inside. As illustrated in Fig. 1, the components removed and recovered by the water separation unit 40 and the carbon dioxide separation unit 50 are returned to a predetermined unit of the system via a water return path 41 and a carbon dioxide return path 51 and are reused. It is illustrated by $H_2O$ and $CO_2$ returned via both return paths 41 and 51, respectively.

As a result, the hydrocarbon production system 100 is established as a carbon closed system that does not substantially release $CO_2$ to the outside of the system.

In Fig. 1, the gas flowing into each unit is illustrated before each unit, and the gas released from the unit is illustrated after each unit.

In the electrolysis reaction unit 10, $H_2O$ and $CO_2$ as starting materials flow in and are electrolyzed internally, $H_2O$ is decomposed into $H_2$ and $O_2$, and some $CO_2$ is decomposed into CO and $O_2$ and released.

The reaction is described as follows.

$$2H_2O \rightarrow 2H_2 + O_2 \qquad \text{(Formula 1)}$$

$$2CO_2 \rightarrow 2CO + O_2 \qquad \text{(Formula 2)}$$

Formulas 1 and 2 are also illustrated in a box illustrating the electrolysis reaction unit 10 of Fig. 1.

In the first catalytic reaction unit 20 (reverse water-gas shift reaction unit), $H_2$ and $CO_2$ flow in, a reverse water-gas shift reaction occurs inside, $CO_2$ becomes CO, $H_2$ becomes $H_2O$, and CO and $H_2O$ are released.

The reaction is described as the following equilibrium reaction, but the reverse water-gas shift reaction is a reaction (reaction proceeding in a direction in which $CO_2$ and $H_2$ react to generate CO and $H_2O$) in which the reaction described by the following formula 3 proceeds to the right.

$$CO_2 + H_2 \Leftrightarrow CO + H_2O \qquad \text{(Formula 3)}$$

Formula 3 is also illustrated in a box illustrating the first catalytic reaction unit 20 (reverse water-gas shift reaction unit) in Fig. 1. A reverse water-gas shift catalyst cat1 used in the reaction is also schematically illustrated in this box.

In the second catalytic reaction unit 30 (hydrocarbon synthesis reaction unit), $H_2$ and CO flow in, and hydrocarbon is synthesized by a catalytic reaction. For example, the reaction in which $CH_4$ is synthesized from CO and $H_2$ is described as the following equilibrium reaction, but the reaction in which $CH_4$ is synthesized from CO and $H_2$ is a reaction (reaction proceeding in a direction in which CO and $H_2$ react to generate $CH_4$ and $H_2O$) in which the reaction described by the following formula 4 proceeds to the right.

$$CO + 3H_2 \Leftrightarrow CH_4 + H_2O \qquad \text{(Formula 4)}$$

Formula 4 is also illustrated in a box illustrating the second catalytic reaction unit 30 (hydrocarbon synthesis reaction unit) in Fig. 1. A hydrocarbon synthesis catalyst cat2 used in the reaction is also schematically illustrated in this box.

Furthermore, the equilibrium reaction of (Formula 3) also occurs at this unit.

Further, depending on the type of catalyst used in the second catalytic reaction unit 30, it is possible to proceed with a Fischer-Tropsch (FT) synthesis reaction or the like. Therefore, various hydrocarbons such as ethane, propane, butane, pentane, hexane, paraffin, and olefinic hydrocarbons can be synthesized from CO and $H_2$.

As will be described later, the inventors have illustrated an example of a catalyst using ruthenium as a catalytically active component of the hydrocarbon synthesis catalyst cat2 disposed in the second catalytic reaction unit 30, but heavy hydrocarbons are also synthesized in a catalyst containing iron, cobalt, or the like as the catalytically active component, and this type of heavy hydrocarbon can be condensed and separated from a transport gas as the temperature decreases.

Therefore, the above-mentioned heavy hydrocarbon separation unit 35 separates the hydrocarbon component separated in this manner.

The $H_2O$ generated in the water separation unit 40 is separated and returned to the upstream side of the electrolysis reaction unit 10 via the water return path 41 (water recycle line).

The $CO_2$ generated in the carbon dioxide separation unit 50 is separated and returned to the upstream side of the electrolysis reaction unit 10 via the carbon dioxide return path 51 (carbon dioxide recycle line).

As a result, in this hydrocarbon production system 100, the hydrocarbon is finally synthesized and can be supplied to the outside.

The above is the outline of the above-mentioned hydrocarbon production system 100, and a configuration of each unit and a role thereof will be described below.

[Electrolysis Reaction Unit]

As illustrated above, the electrolysis reaction unit 10 decomposes $H_2O$ and $CO_2$ that flow in by consuming electric power supplied according to the above formulas 1 and 2.

Fig. 2 schematically illustrates a cross-sectional structure of the electrolysis reaction unit 10.

Fig. 2 illustrates an electrolysis cell unit U which is stacked in multiple to form an electrolysis stack (not illustrated). The electrolysis cell unit U includes an electrolysis cell 1, and the electrolysis cell 1 includes an electrode layer 2 on one surface of an electrolyte layer 1a and a counter electrode layer 3 on the other surface thereof. The electrode layer 2 serves as a cathode in the electrolysis cell 1, and the counter electrode layer 3 serves as an anode. Incidentally, this electrolysis cell unit U is supported by a metal support 4. Here, a case where a solid oxide type electrolysis cell is used as the electrolysis cell 1 is illustrated.

The electrolyte layer 1a can be formed in the state of a thin film having a thickness of 10 μm or less. As a constituent material of the electrolyte layer 1a, YSZ (yttria-stabilized zirconia), SSZ (scandia-stabilized zirconia), GDC (gadolinium-doped ceria), YDC (yttrium-doped ceria), SDC (samarium-doped ceria), and LSGM (strontium/magnesium-added lanthanum gallate), or the like can be used. In particular, zirconia-based ceramics are preferably used.

Preferably, the electrolyte layer 1a is formed by a low-temperature calcination method (for example, a wet method using a calcination treatment in a low temperature range that does not carry out a calcination treatment in a high temperature range exceeding 1100°C), a spray coating method (thermal spraying method, aerosol deposition method, aerosol gas deposition method, a powder jet deposition method, particle jet deposition method, cold spray method, or the like), a PVD method (sputtering method, a pulse laser deposition method, or the like), a CVD method, or the like. These film forming processes that can be used in a low temperature range provide an electrolyte layer 1a that is dense and has high airtightness and gas barrier properties without using calcination in a high temperature range exceeding, for example, 1100°C. Therefore, damage to the metal support 4 can be suppressed, element mutual diffusion between the metal support 4 and the electrode layer 2 can be suppressed, and an electrolysis cell unit U having excellent performance and durability can be realized. In particular, it is preferable to use the low-temperature calcination method, the spray coating method, or the like because a low-cost element can be realized. Further, it is more preferable to use the spray coating method because the electrolyte layer 1a, which is dense and has high airtightness and gas barrier property, can be easily obtained in a low temperature range.

Further, the electrolyte layer 1a is densely configured in order to prevent the gas leak and exhibit high ionic conductivity. A density of the electrolyte layer 1a is preferably 90% or more, more preferably 95% or more, and further preferably 98% or more. When the electrolyte layer 1a is a uniform layer, the density is preferably 95% or more, and more preferably 98% or more. When the electrolyte layer 1a includes a plurality of layers, it is preferable that at least a portion of the electrolyte layer 1a includes a layer (dense electrolyte layer) having a density of 98% or more, and it is more preferable to include a layer (dense electrolyte layer) having a density of 99% or more. In a case where the dense electrolyte layer is included in a portion of the electrolyte layer 1a, even when the electrolyte layer 1a includes a plurality of layers, it is possible to easily form the electrolyte layer 1a that is dense and has high airtightness and gas barrier property.

The electrode layer 2 can be provided in a thin layer on the front surface of the metal support 4 and in a region larger than a region where holes 4a are provided. In the case of a thin layer, a thickness thereof can be, for example, about 1 μm to 100 μm, preferably 5 μm to 50 μm. With such a thickness, it is possible to secure sufficient electrode performance while reducing the amount of expensive electrode layer material used to reduce costs. The entire region provided with the holes (through holes) 4a is covered with the electrode layer 2. That is, the hole 4a is formed inside the region of the metal support 4 where the electrode layer 2 is formed. In other words, all the holes 4a are provided facing the electrode layer 2.

As the constituent material of the electrode layer 2, for example, a composite material such as NiO-GDC, Ni-GDC, NiO-YSZ, Ni-YSZ, CuO-$CeO_2$, Cu-$CeO_2$ can be used. In these examples, GDC, YSZ, and $CeO_2$ can be referred to as aggregates of the composite material. Preferably, the electrode layer 2 is formed by a low-temperature calcination method (for example, a wet method using a calcination treatment in a low temperature range that does not carry out a calcination treatment in a high temperature range exceeding 1100°C), a spray coating method (thermal spraying method, aerosol deposition method, aerosol gas deposition method, a powder jet deposition method, particle jet deposition method, cold spray method, or the like), a PVD method (sputtering method, a pulse laser deposition method, or the like), a CVD method, or the like. These processes that can be used in the low temperature range provide an improved electrode layer 2 without using, for example, calcination in a high temperature range exceeding 1100°C. Therefore, preferably, the metal support 4 is not damaged, element mutual diffusion between the metal support 4 and the electrode layer 2 can be suppressed, and an electrochemical element having excellent durability can be realized. Further, it is more preferable to use the low-temperature calcination method because the handling of the raw material becomes easy.

The counter electrode layer 3 can be formed in a thin layer on the surface opposite to the electrode layer 2 of the electrolyte layer 1a. In the case of a thin layer, a thickness thereof can be, for example, about 1 $\mu$m to 100 $\mu$m, preferably 5 $\mu$m to 50 $\mu$m. With such a thickness, it is possible to secure sufficient electrode performance while reducing the amount of expensive counter electrode layer material used to reduce costs. As the material of the counter electrode layer 3, for example, a composite oxide such as LSCF or LSM, a ceria-based oxide, or a mixture thereof can be used. In particular, it is preferable that the counter electrode layer 3 contains a perovskite-type oxide containing two or more kinds of elements selected from the group consisting of La, Sr, Sm, Mn, Co, and Fe.

The electrolyte layer 1a, the electrode layer 2, and the counter electrode layer 3 are formed as a thin film as described later, and the inventor calls this thin layer forming.

As illustrated above, the electrolysis cell unit U has a metal support type, includes a metal support 4 as a support for the electrode layer 2, and a supply path forming member 5 for forming a U-shaped electrode layer-side gas supply path 5a is provided on a side opposite to the electrode layer 2 in a state where the metal support 4 is interposed therebetween. Further, the metal support 4 is provided with a large number of holes 4a penetrating the front and back surfaces. The gas ($H_2O$ and $CO_2$) supplied through the electrode layer-side gas supply path 5a is subject to electrolysis and is supplied to the electrode layer 2 through a large number of holes 4a. Further, the generated gas ($H_2$, CO) is discharged from the hole 4a.

Meanwhile, also on the counter electrode layer 3 side, a supply path forming member 6 for forming a counter electrode layer-side gas supply path 6a is provided. As illustrated in Fig. 2, the supply path forming member 6 is provided with many grooves on the counter electrode layer 3 side and is configured to supply a transport gas g2 (for example, air) to the counter electrode layer-side gas supply path 6a.

The metal support 4 supports the electrode layer 2, the electrolyte layer 1a, and the counter electrode layer 3 and serves as a support for maintaining the strength of the electrolysis cell 1 and the electrolysis cell unit U as a whole. In this example, the plate-shaped metal support 4 is used as the metal support, but other shapes such as a box shape and a cylindrical shape are also possible.

The metal support 4 may have sufficient strength to form the electrolysis cell unit U as a support, and for example, can use a support having a thickness of about 0.1 mm to 2 mm, preferably about 0.1 mm to 1 mm, and more preferably about 0.1 mm to 0.5 mm. In the present embodiment, the support is made of metal, but ceramics can also be used, for example.

The metal support 4 has, for example, the plurality of holes 4a provided so as to penetrate the front surface and the back surface of the metal plate. For example, the hole 4a can be provided in the metal support 4 by mechanical, chemical, or optical drilling. The hole 4a has a function of allowing gas to pass from the back surface to the front surface of the metal support 4. The hole 4a may be provided so as to be inclined in a gas flow direction (the front and back directions of the paper surface in Fig. 2).

By using a ferrite-based stainless steel material (an example of an Fe-Cr-based alloy) as a material of a base material of the metal support 4, a thermal expansion coefficient of the metal support 4 can be made close to those of YSZ (yttria-stabilized zirconia), GDC (gadolinium-doped ceria, also referred to as CGO), and the like used as materials for the electrode layer 2 and the electrolyte layer 1a. Therefore, the electrolysis cell unit U is less likely to be damaged even when the low temperature and high temperature cycles are repeated. Therefore, it is preferable because the electrolysis cell unit U having excellent long-term durability can be realized.

The same material as that of the metal support 4 can be used for the supply path forming members 5 and 6 of the electrolysis cell unit U, and the thickness thereof can be substantially the same.

Although the metal support 4 and both supply path forming members 5 and 6 have conductivity, they are gastightly configured to function as a separator for separating the supply paths 5a and 6a.

In the electrolysis cell unit U having the above configuration, in an electrolysis operation, DC power is supplied between the pair of electrode layers 2 and 3 provided with the electrolyte layer 1a interposed therebetween. In the present embodiment, as illustrated in Fig. 2, the case where the electrode layer 2 side is negative and the counter electrode layer 3 side is positive is illustrated. Depending on the configuration of the electrolysis cell unit U, the electrode layer 2 side may be positive and the counter electrode layer 3 side may be negative.

Then, $H_2O$ and $CO_2$, which are gases to be electrolyzed, are supplied to the electrode layer 2, and the transport gas g2 is supplied to the counter electrode layer side. Therefore, the reactions illustrated in the formulas 1 and 2 can be caused in the electrolysis cell 1 and the decomposed gas can be taken out. Here, regarding the supply of $H_2O$, either water or water vapor may be used, or both of them may be used. Therefore, in the present invention, an electrolysis cell device is constructed which includes at least the electrolysis cell unit U, the electrolysis raw material supply unit that supplies water and/or water vapor and carbon dioxide to the electrolysis cell unit U, and the power supply unit that supplies electric power.

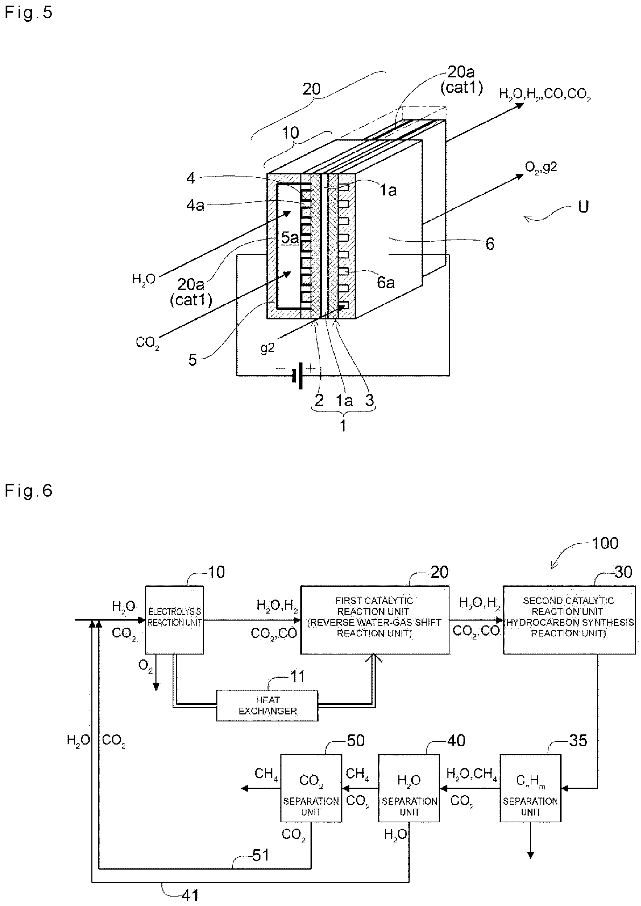

The supplied gas ($H_2O$, $CO_2$) and the released gas ($H_2O$, $H_2$, CO, $O_2$, $CO_2$) in the electrolysis reaction are illustrated above and below the electrolysis cell unit U in Fig. 2. However, this is for ease of understanding, and in fact, the above-mentioned electrode layer-side gas supply path 5a and counter electrode layer-side gas supply path 6a are formed so as to extend in the front and back directions of the paper surface of Fig. 2, and for example, the gas ($H_2O$, $CO_2$) on the supply side described on an upper side of the electrolysis cell unit U in Fig. 2 can be recovered from the front side of the paper surface, and the gas ($H_2O$, $H_2$, CO, $O_2$, $CO_2$) on the release side described on the lower side of the electrolysis cell 1 can be recovered from the back side of the paper surface (refer to Fig. 5 described later). In addition, in order to smoothly perform the discharge of $O_2$ generated in the electrolysis reaction, for example, the transport gas g2 such as air can flow through the electrolysis cell unit U.

When $H_2O$ and $CO_2$ are supplied to the electrolysis reaction unit 10 to carry out the electrolysis, $H_2O$ has a lower electrolysis voltage than $CO_2$ and is easily electrolyzed. Therefore, when $H_2O$ and $CO_2$ having the same amount are temporarily supplied to the electrolysis reaction unit 10 and the electrolysis reaction is carried out, the $H_2$ concentration tends to be higher than the CO concentration at the outlet of the electrolysis reaction unit 10, and unreacted $CO_2$ tends to remain.

[First Catalytic Reaction Unit (Reverse Water-Gas Shift Reaction Unit)]

As illustrated above, the first catalytic reaction unit 20 (reverse water-gas shift reaction unit) causes a reverse water-gas shift reaction, converts $CO_2$ into CO using the supplied $H_2$, and converts $H_2$ into $H_2O$. That is, in the electrolysis reaction unit 10 that supplies $H_2O$ and $CO_2$ to electrolyze, the remaining $CO_2$ that is not decomposed is converted into CO.

The reaction here is as illustrated by the formula 3, but this reaction is an endothermic reaction and is an equilibrium reaction according to the reaction temperature conditions. As a result, it is preferable that the catalyst is capable of causing the reaction represented by the formula 3 on the high temperature side (for example, 600°C to 800°C) as much as possible.

In the description of the catalyst in the present specification, a component having activity as a catalyst may be referred to as a "catalytically active component", and a carrying body supporting the catalytically active component may be referred to as a "carrier (also referred to as "support" in the catalyst).

The inventors examined various combinations of catalytically active components and carriers as described later, and found that a specific combination was suitable.

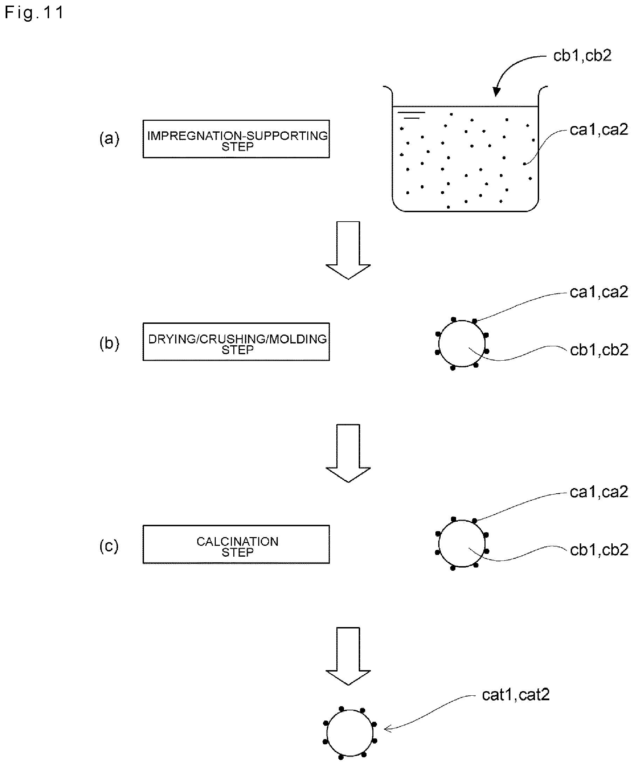

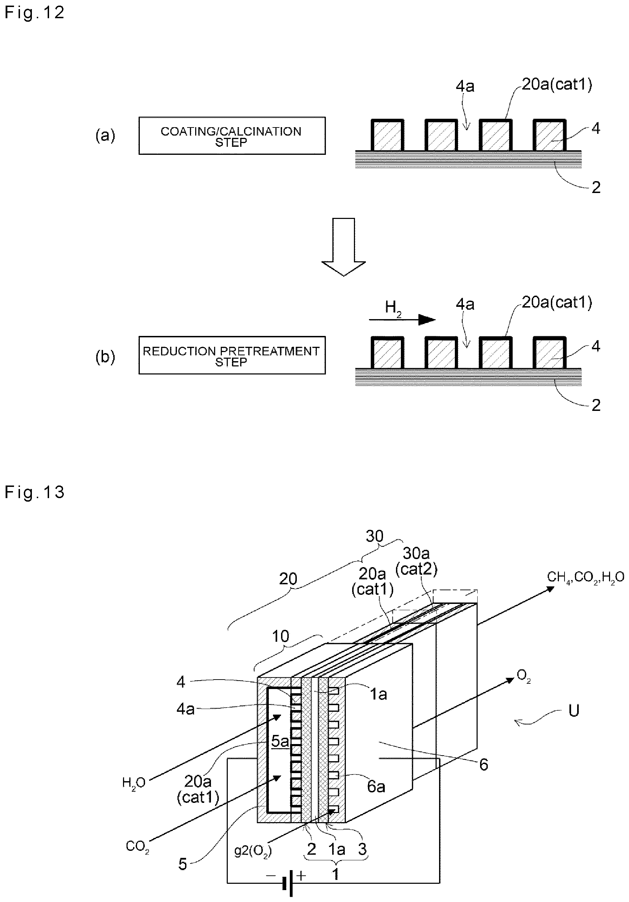

In a production of this type of catalyst, by executing an impregnation-supporting step of immersing the carrier (metal oxide) in a solution containing a catalytically active component (metal component), taking out the carrier, drying and heat-treating the carrier, it is possible to easily obtain a carrier-supported catalyst (impregnated supported product) in which the catalytically active component is distributed on the surface of the carrier. This heat treatment is a calcination treatment. The preparation and use of the catalyst will be described with reference to Figs. 11 and 12.

A preparation method described here is the same except that the starting material is different in the combination of various catalytically active components and carriers. Fig. 11 illustrates examples of the reverse water-gas shift catalyst cat1 and the hydrocarbon synthesis catalyst cat2 according to the present invention. In Fig. 11, the catalytically active component of the reverse water-gas shift catalyst cat1 is referred to as ca1, and the carrier thereof is referred to as cb1. Meanwhile, regarding the hydrocarbon synthesis catalyst cat2, the catalytically active component thereof is ca2 and the carrier thereof is cb2.

As illustrated in Fig. 11, in the catalyst preparation, after an impregnation-supporting step (a) of obtaining an aqueous solution of a compound containing a metal component (which is a metal catalyst) to be the catalytically active components ca1 and ca2, inputting the carriers cb1 and cb2 into the aqueous solution, and carrying out stirring and impregnation is executed, a drying/crushing/molding step (b) or the like of carrying out evaporative drying, drying, and crushing and molding is executed, and thereafter, a calcination step (c) of calcinating an obtained molded product in the air is executed, and thus, the target product (cat1, cat2) can be obtained. Therefore, this form of catalyst is also referred to as an impregnation-supported catalyst. In addition, the step (b) can be performed up to the drying step, and a crushing/molding treatment can be performed after the calcination step (c). In addition, the impregnated supported product (product before being subjected to the calcination step (c), regardless of whether or not the step such as crushing/molding is performed) obtained by the impregnation step is referred to as a precursor in the present invention.

In this case, as illustrated in the example of the reverse water-gas shift catalyst cat1 in Fig. 12, the catalyst can be applied to a portion where the catalyst is used and calcined. Fig. 12(a) illustrates a coating/calcination step in which the reverse water-gas shift catalyst cat1 is applied to the metal support 4 in which the holes 4a are perforated to form a coating layer 20a, and the coating layer 20a is calcinated. Fig. 12(b) illustrates a reduction pretreatment step in which $H_2$ flows to carry out a reduction pretreatment before using the reverse water-gas shift catalyst cat1.

When the calcination treatment is carried out in air, the supported catalytically active components ca1 and ca2 are in a state where a part or all of them are oxidized. Before using the catalyst, a so-called reduction pretreatment may be carried out to reduce the catalytically active component in an oxidized state to sufficiently enhance the activity. Fig. 12(b) illustrates a state in which a reducing gas (typically $H_2$) is circulated on the surface of the catalyst to carry out the reduction pretreatment.

(Catalyst Used)

As the reverse water-gas shift catalyst cat1 which is a catalyst used for the first catalytic reaction unit 20, the inventors have selected a catalyst that satisfies the following requirements.

The reverse water-gas shift catalyst cat1 is obtained by calcinating a precursor obtained by adding at least a nickel component to alumina at a temperature of 500°C or higher.

As described above, the reverse water-gas shift catalyst cat1 is produced by performing an impregnation-supporting step of impregnating a carrier cb1 containing alumina as a main component with nickel as a catalytically active component ca1 to be supported on the carrier cb1 and calcinating the precursor obtained in the impregnation-supporting step at a temperature of 500°C or higher.

Here, since the strength of the catalyst cat1 can be increased, a ratio of the carrier cb1 to the entire catalyst is preferably 55% by weight or more, more preferably 60% by weight or more, and further preferably 65% by weight or more. Further, an upper limit of this ratio can be, for example, 99.5% by weight, but when the upper limit is more than this, the catalytically active component ca1 cannot be sufficiently supported, and it may be difficult to obtain the effect as the reverse water-gas shift catalyst.

Hereinafter, test results of Examples, Comparative Examples, Reference Examples in the case where $Al_2O_3$ (alumina) is the carrier cb1 and the catalytically active component ca1 is variously changed as the reverse water-gas shift catalyst cat1 used for the first catalytic reaction unit 20 will be described.

The catalytically active component ca1 was Ni, Fe, and Pt (platinum).

(Catalyst Preparation)

In preparing the reverse water-gas shift catalyst cat1, an aqueous solution is obtained by quantifying and dissolving a water-soluble nickel compound (nickel nitrate, nickel chloride, nickel sulfate, nickel ammonium sulfate, nickel acetate, nickel oxalate, nickel citrate, or the like) and a water-soluble iron compound (iron nitrate, iron chloride, iron sulfate, ammonium iron sulfate, iron acetate, iron oxalate, iron citrate, or the like) according to the composition of the target catalyst. A predetermined amount of carrier powder ($Al_2O_3$) is input to the aqueous solution, stirred and impregnated, then evaporated to dryness, dried, then crushed and molded, and then calcinated in air. This impregnation is the "impregnation-supporting step" referred to in the present invention, and the result is the "impregnated supported product".

The catalysts of the following Examples and Comparative Examples were prepared using nickel nitrate hexahydrate and iron nitrate nonahydrate, respectively, and a metal supported amount of nickel was 9% by weight, and a metal supported amount of iron was 9% by weight. In addition, the catalyst using Pt in the following Reference Example was prepared using tetraammineplatinum hydroxide, and a supported amount of platinum was 1% by weight.

In the above catalyst preparation, evaporation to dryness and drying can be carried out in a generally used temperature range, and were prepared at 80°C and 80°C, respectively. Further, the calcination temperature was variously changed such as 450°C (Comparative Example 1, Reference Example 1), 600°C (Example 1, Comparative Example 2), 800°C (Example 2, Comparative Example 3), 1000°C (Example 3), 1200°C°C (Example 4) and examined.

Table 1 illustrates the reverse water-gas shift catalysts cat1 of Examples (1 to 4), Comparative Examples (1 to 3), and Reference Example (1) prepared.

A horizontal axis represents a type of the carrier cb1, a calcination temperature (°C), a CO adsorption amount (mL(N)/g), and a BET surface area ($m^2/g$).

Regarding the CO adsorption amount, the CO adsorption amount was measured by a CO pulse adsorption method after the catalyst was subjected to a reduction pretreatment at 350°C for 1 hour under a hydrogen atmosphere.

[Table 1]

| Catalyst | | Carrier | Calcination temperature(°C) | CO adsorption amount (Nml/g) | BET surface area ($m^2/g$) |
|---|---|---|---|---|---|
| Example 1 | $Ni/Al_2O_3$ | $Al_2O_3$ | 600 | 0.44 | 103.4 |

(continued)

| Catalyst | | Carrier | Calcination temperature(°C) | CO adsorption amount (Nml/g) | BET surface area (m²/g) |
|---|---|---|---|---|---|
| Example 2 | $Ni/Al_2O_3$ | $Al_2O_3$ | 800 | 0.04 | 100.8 |
| Example 3 | $Ni/Al_2O_3$ | $Al_2O_3$ | 1000 | 0.08 | 83.6 |
| Example 4 | $Ni/Al_2O_3$ | $Al_2O_3$ | 1200 | 0.11 | 14.5 |
| Comparative Example 1 | $Ni/Al_2O_3$ | $Al_2O_3$ | 450 | 0.49 | 107.5 |
| Comparative Example 2 | $Fe/Al_2O_3$ | $Al_2O_3$ | 600 | 0.15 | 96.0 |
| Comparative Example 3 | $Fe/Al_2O_3$ | $Al_2O_3$ | 800 | 0.14 | 83.5 |
| Reference Example 1 | $Pt/Al_2O_3$ | $Al_2O_3$ | 450 | 1.85 | 97.8 |

(Catalytic Activity Test)

In the catalytic activity test, a mixed gas containing 50% $H_2$-50% $CO_2$ was used as a reaction gas, and the reaction temperature was changed from 600°C to 800°C in increments of 50°C under the conditions in which GHSV was 10000/h.

Before conducting the catalytic activity test, the reduction pretreatment of the catalyst was carried out at 600°C while flowing a hydrogen gas through the catalyst layer.

As the test results, the $CO_2$ conversion rate (%), the CO concentration (%) at the outlet of the reaction unit, and the $CH_4$ concentration (%) are illustrated in Table 2.

The $CO_2$ conversion rate (%) was calculated according to the following formula based on a gas analysis result at the outlet of the catalyst layer.

$$[CH_4 \text{ concentration}] + [CO \text{ concentration}]/([CH_4 \text{ concentration}] + [CO \text{ concentration}]$$

$$+ [CO_2 \text{ concentration}])$$

As illustrated above, in the reverse water-gas shift catalyst cat1 used in the first catalytic reaction unit 20 (reverse water-gas shift reaction unit), it is desirable that the $CO_2$ conversion rate (%) on the high temperature side (for example, around 600 to 800°C) is high.

[Table 2]

| Catalyst | | | Reaction temperature (°C) | | | | |
|---|---|---|---|---|---|---|---|
| | | | 600 | 650 | 700 | 750 | 800 |
| Example 1 | $Ni/Al_2O_3$ | $CO_2$ conversion rate (%) | 32.2 | 38.0 | 40.9 | 42.6 | 44.9 |
| | | Outlet CO concentration (%) | 17.8 | 23.5 | 25.4 | 27.0 | 28.8 |
| | | Outlet $CH_4$ concentration (%) | 3.6 | 1.0 | 0.3 | 0.1 | 0.1 |
| Example 2 | $Ni/Al_2O_3$ | $CO_2$ conversion rate (%) | 33.8 | 39.5 | 44.0 | 45.6 | 47.6 |
| | | Outlet CO concentration (%) | 19.3 | 25.0 | 27.5 | 29.7 | 31.5 |
| | | Outlet $CH_4$ concentration (%) | 3.4 | 0.7 | 0.2 | 0.0 | 0.0 |

(continued)

| Catalyst | | | Reaction temperature (°C) | | | | |
|---|---|---|---|---|---|---|---|
| | | | 600 | 650 | 700 | 750 | 800 |
| Example 3 | $Ni/Al_2O_3$ | $CO_2$ conversion rate (%) | 35.0 | 40.0 | 42.3 | 44.7 | 45.8 |
| | | Outlet CO concentration (%) | 19.7 | 24.4 | 27.3 | 28.9 | 30.3 |
| | | Outlet $CH_4$ concentration (%) | 3.5 | 0.7 | 0.2 | 0.1 | 0.0 |
| Example 4 | $Ni/Al_2O_3$ | $CO_2$ conversion rate (%) | 34.5 | 40.0 | 43.1 | 45.5 | 46.7 |
| | | Outlet CO concentration (%) | 19.4 | 24.7 | 27.4 | 29.5 | 30.8 |
| | | Outlet $CH_4$ concentration (%) | 3.4 | 0.9 | 0.2 | 0.1 | 0.0 |
| Comparative Example 1 | $Ni/Al_2O_3$ | $CO_2$ conversion rate (%) | 31.2 | 35.5 | 36.9 | 38.2 | 40.0 |
| | | Outlet CO concentration (%) | 16.1 | 20.4 | 22.5 | 23.7 | 24.8 |
| | | Outlet $CH_4$ concentration (%) | 4.1 | 1.5 | 0.8 | 0.5 | 0.4 |
| Comparative Example 2 | $Fe/Al_2O_3$ | $CO_2$ conversion rate (%) | 24.8 | 29.0 | 35.3 | 40.6 | 45.3 |
| | | Outlet CO concentration (%) | 14.0 | 16.8 | 20.8 | 25.3 | 29.5 |
| | | Outlet $CH_4$ concentration (%) | 0.0 | 0.0 | 0.0 | 0.0 | 0.0 |
| Comparative Example 3 | $Fe/Al_2O_3$ | $CO_2$ conversion rate (%) | 22.2 | 25.2 | 32.7 | 38.9 | 43.9 |
| | | Outlet CO concentration (%) | 13.4 | 15.1 | 20.0 | 24.5 | 29.1 |
| | | Outlet $CH_4$ concentration (%) | 0.0 | 0.0 | 0.0 | 0.0 | 0.0 |
| Reference Example 1 | $Pt/Al_2O_3$ | $CO_2$ conversion rate (%) | 35.5 | 41.1 | 44.7 | 47.5 | 49.6 |
| | | Outlet CO concentration (%) | 19.4 | 24.6 | 27.6 | 29.7 | 31.2 |
| | | Outlet $CH_4$ concentration (%) | 3.2 | 0.8 | 0.2 | 0.1 | 0.0 |
| Reference (equilibrium value) | | $CO_2$ conversion rate (%) | 34.6 | 39.9 | 43.6 | 46.4 | 49.0 |

For reference, an equilibrium value (calculated value) of the $CO_2$ conversion rate under the experimental conditions is illustrated in Table 2.

Fig. 3 illustrates the results of the reverse water-gas shift catalyst cat1 (nickel/alumina catalyst) of interest in the present invention when only the calcination temperature was varied.

In Fig. 3, a vertical axis is the $CO_2$ conversion rate (%) and a horizontal axis is a reaction temperature (°C).

Nickel/Alumina Catalyst

As can be seen from Fig. 3, Examples 1 to 3, and Comparative Example 1, it was found that while activity of the catalyst calcinated at 450°C was low, when the calcination temperature was raised above 500°C, the catalytic activity increased step by step, and a $CO_2$ conversion rate reached the vicinity of an equilibrium value (calculated value) in particularly the catalyst calcinated at 800°C or 1000°C. It was also found that even with a catalyst whose calcination temperature was raised to 1200°C, there was no significant decrease in catalytic activity. Then, it was found that the reverse water-gas shift reaction can carried out with the $CO_2$ conversion rate (%) equal to that of the Pt catalyst, which is highly active but very expensive, illustrated in Reference Example 1.

Incidentally, the nickel/alumina catalyst whose activity was remarkably increased had the $CO_2$ adsorption amount of 0.45 (mL(N)/g) or less.

In addition, nickel/alumina catalysts with different calcination temperatures were subjected to X-ray diffraction tests. The constituents of the catalyst were confirmed. Moreover, an average particle size was confirmed.

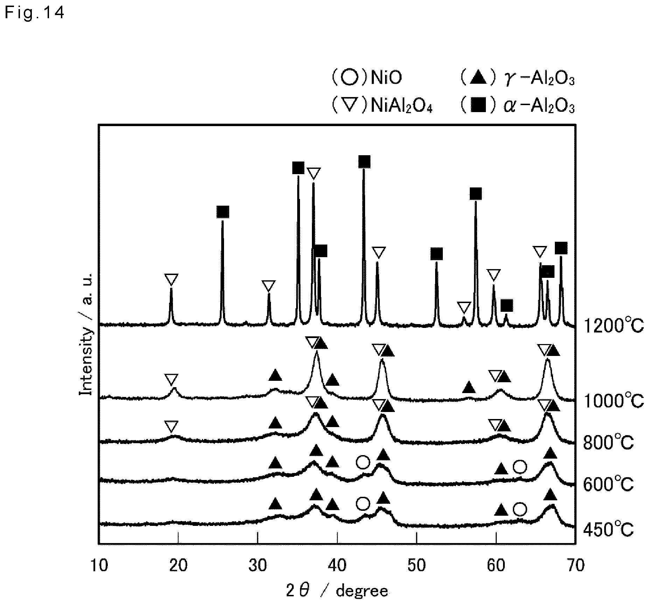

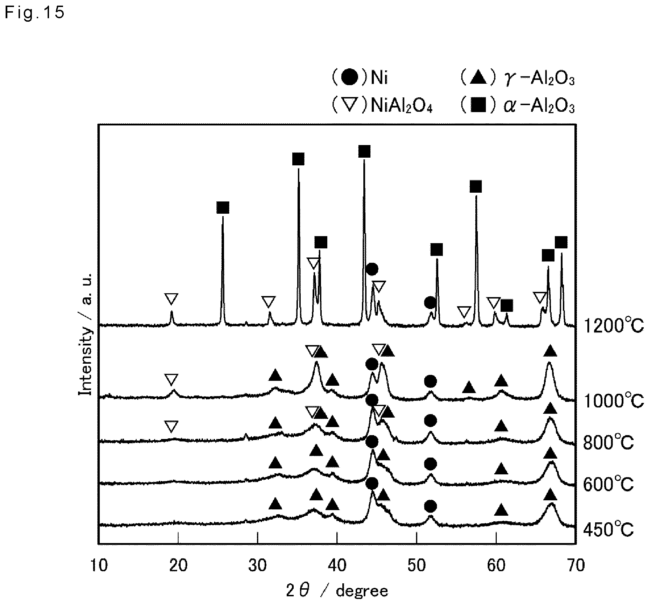

Figs. 14 and 15 illustrate results of the X-ray diffraction test. Fig. 14 illustrates results before using the nickel/alumina catalyst and Fig. 15 illustrates results after using the catalyst continuously for 90 hours. In Figs. 14 and 15, white circles correspond to NiO, black upward triangles correspond to $\gamma$-$Al_2O_3$ ($\gamma$-alumina), white downward triangles

correspond to $NiAl_2O_4$, and black squares correspond to $\alpha$-$Al_2O_3$ ($\alpha$-alumina).

From these results, it can be seen that when the calcination temperature is 800°C or higher, a $NiAl_2O_4$ phase is generated as a crystalline phase in the nickel/alumina catalyst. A $NiAl_2O_4$ peak is significant at 1200°C calcination, but decreases after use. With respect to alumina, $\alpha$-alumina is a main component at 1000°C or lower and $\alpha$-alumina turns into $\gamma$-alumina when calcinated at 1200°C or higher.

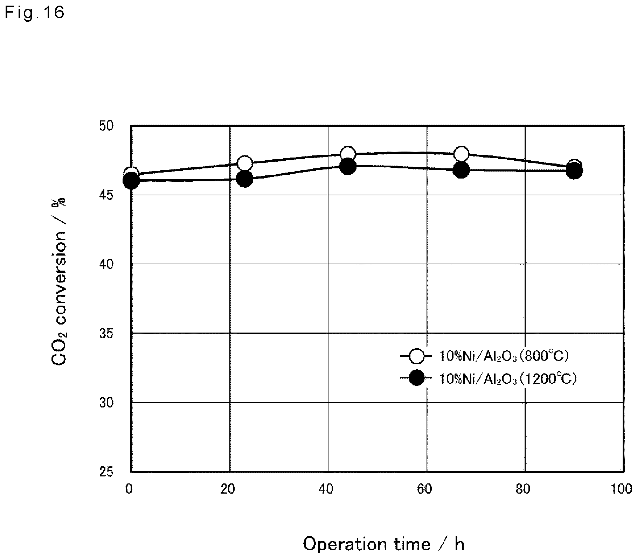

Fig. 16 is a graph illustrating a relationship between an operation time (h) and a conversion rate (%) for the 800°C calcinated product (white circles) and the 1200°C calcinated product (black circles), which were determined to be preferable in the present invention. As illustrated in Fig. 16, even when the continuous operation time is extended, the conversion rate does not change significantly. That is, it was confirmed that continuous use was possible without deterioration in performance.

Furthermore, the average particle size of Ni contained in the catalyst was confirmed for nickel/alumina catalysts with different calcination temperatures of 450°C, 600°C, 800°C, 1000°C, and 1200°C. The average particle size of Ni was calculated by using a half width of the diffraction peak corresponding to Ni obtained in the X-ray diffraction test and a Scherrer's formula. As a result, Ni calcinated at 450°C had the average particle size of 6.5 nm or less, and Ni calcinated at a temperature higher than 600°C had the average particle size in a range of 9 nm or more to 10 to 30 nm. The average particle size tended to increase as the operation time increased. As a result, the nickel/alumina catalyst in which the average particle size of Ni is 10 nm or more is suitable as the reverse water-gas shift catalyst of the present invention.

Furthermore, a catalyst containing a $NiAl_2O_4$ phase as a crystalline phase and having any of the following properties (1) to (3) is also suitable as the reverse water-gas shift catalyst of the present invention.

    (1) The CO adsorption amount per 1 g of catalyst is 0.45 mL or less.
    (2) The average crystal particle size of nickel is 10 nm or more.
    (3) The BET surface area is 105 $m^2$ or less.

Iron/Alumina Catalyst

As can be seen from Comparative Examples 2 and 3, unlike the nickel/alumina catalyst, in the iron/alumina catalyst, when the calcination temperature is raised from 600°C to 800°C, the catalytic activity decreases. In particular, it can be seen that the catalytic activity is generally lower than that of the nickel/alumina catalyst, in a relatively low reaction temperature range of 600°C to 750°C.

Usefulness as Reverse Water-Gas Shift Catalyst

From the above, as the reverse water-gas shift catalyst cat1 used in the reverse water-gas shift reaction unit 20, it is possible to adopt the reverse water-gas shift catalyst in which the precursor obtained by adding at least a nickel component to alumina is calcinated at a temperature of 500°C or higher.

In this case, specifically, it is possible to obtain the reverse water-gas shift catalyst by executing the impregnation-supporting step of impregnating the carrier containing alumina as the main component with nickel to be supported on the carrier and calcinating the precursor obtained in the impregnation-supporting step at the temperature of 500°C or higher.

By using the reverse water-gas shift catalyst cat1 in the first catalytic reaction unit 20 (reverse water-gas shift reaction unit), the reverse water-gas shift reaction can be carried out at around 600 to 1000°C with the $CO_2$ conversion rate (%) about equal to that of the Pt catalyst, which is highly active but very expensive.

Since the test of this example was carried out under a very high GHSV condition of 10000/h, by reducing the GHSV to less than 10000/h, that is, by increasing the amount of catalyst used with respect to the amount of gas to be treated, it is possible to carry out the reverse water-gas shift reaction at a higher $CO_2$ conversion rate (%).

[Combination of Electrolysis Reaction Unit and Reverse Water-Gas Shift Reaction Unit]

In the description so far, according to the system configuration illustrated in Fig. 1, the electrolysis reaction unit 10 and the reverse water-gas shift reaction unit 20 are individually provided in the order described along the flow direction of the gas.

The reaction of the electrolysis reaction unit 10 is an exothermic reaction depending on the reaction conditions, and the reaction of the reverse water-gas shift reaction unit 20 is an endothermic reaction. Therefore, thermal efficiency of the system can be improved by integrating the two reaction units 10 and 20. In this way, Fig. 4 illustrates a configuration in which the two reaction units 10 and 20 are combined and integrated, and the integration is illustrated to surround both units. In addition, a reaction when integrated in the same box in this way is illustrated. Basically, the above-mentioned formulas 1, 2, and 3 are carried out. In a case where the electrolysis reaction unit 10 and the reverse water-gas shift reaction unit 20 are combined and integrated, preferably, when the units are surrounded together by a heat insulating member, heat can be efficiently transferred between the electrolysis reaction unit 10 and the reverse water-gas shift reaction unit 20. Further, in order to transfer the heat generated in the electrolysis reaction unit 10 to the reverse water-gas shift reaction unit 20, the electrolysis reaction unit 10 and the reverse water-gas shift reaction unit 20 may be connected using a heat transfer member.

[Electrolysis Cell Unit Equipped with Both Electrolysis Reaction Unit and Reverse Water-Gas Shift Reaction Unit]

Based on the above concept, it is preferable to provide the reverse water-gas shift reaction unit 20 in the electrolysis cell unit U which is the electrolysis reaction unit 10. This is because when a solid oxide type electrolysis cell that operates at around 600 to 800°C is used as the electrolysis cell 1, in the reverse water-gas shift catalyst cat1 of the present application which can obtain high activity at around 600 to 800°C, the electrolysis reaction unit 10 and the reverse water-gas shift reaction unit 20 can be used in the same temperature range.

In this case as well, it is sufficient that the gas that has passed through the electrolysis reaction unit 10 is guided to the reverse water-gas shift reaction unit 20 to generate the reverse water-gas shift reaction.

Fig. 5 illustrates an electrolysis cell unit U provided with such a reverse water-gas shift reaction unit 20. Fig. 5 is a diagram illustrating the electrolysis cell unit U illustrated in cross section in Fig. 2 including the flow direction of the gas.

As illustrated in Fig. 5, the cross sections of the electrolysis cell unit U are basically the same.

That is, the electrolysis cell unit U also includes the electrolysis cell 1 in which the electrode layer 2 and the counter electrode layer 3 are formed with the electrolyte layer 1a interposed therebetween, the metal support 4 which functions as a support thereof and also functions as a separator, and the supply path forming members 5 and 6, and the electrode layer-side gas supply path 5a and the counter electrode layer-side gas supply path 6a are formed in the electrolysis cell unit U. More specifically, as can be seen from Fig. 4, when the metal support 4 is viewed in the flow direction of gas, the holes 4a are provided in the portion corresponding to the electrolysis cell 1, but the hole is not provided on the downstream side of the electrode layer 2. Therefore, the metal support 4 corresponds to a separator which effectively separates the gas which is supplied to the electrode layer 2 and released from the electrode layer 2, and the gas which is supplied to the counter electrode layer 3 gas and is released from the counter electrode layer 3.

However, in this example, the reverse water-gas shift catalyst cat1 described above is applied to an inner surface (supply path-side inner surface of supply path forming member 5, surface of the metal support 4 opposite to surface on which electrode layer 2 is formed, and surfaces of the plurality of holes 4a) of the electrode layer-side gas supply path 5a. A coating layer 20a is illustrated by a thick solid line. Further, the electrode layer-side gas supply path 5a extends beyond the electrolysis reaction unit 10, and the coating layer 20a is also provided on the extension side.

As a result, the electrode layer-side gas supply path 5a of the electrolysis cell unit U is a discharge path for discharging at least $H_2$ generated in the electrode layer 2, and the electrolysis cell unit U is integrally provided with the electrolysis reaction unit 10 and the reverse water-gas shift reaction unit 20.

In this configuration, the metal support 4 acts as a separator that separates $H_2$ generated in the electrode layer 2 and $O_2$ generated in the counter electrode layer 3, and at least a portion of the separator on the discharge path side of $H_2$ is reverse water-gas shift reaction unit 20.

By stacking the electrolysis cell units U configured in this way in a right-left direction of Figs. 2 and 5, a large number of electrolysis cell units U are stacked, and it is possible to form a electrolysis cell module (not illustrated) in which the electrolysis cell units are electrically connected to each other. Of course, a useful gas generated can be obtained over multiple layers.

In comparison between the electrolysis cell unit U that does not house the reverse water-gas shift catalyst cat1 in the electrode layer-side gas supply path 5a (which serves as a discharge path for the electrolyzed gas) and the electrolysis cell unit U that houses the reverse water-gas shift catalyst cat1, at an outlet, a hydrogen/carbon monoxide ($[H_2/CO]$) ratio is about 10 to about 5, which is preferable because the amount of CO required for hydrocarbon synthesis can be secured. In addition, since thermal efficiency of the hydrocarbon production system 100 can be improved by adopting a methanation reaction of CO rather than a methanation reaction of $CO_2$, by combining the reaction of the electrolysis reaction unit 10 and the reaction of the reverse water-gas shift reaction unit 20, the amount of CO can be secured, which is preferable. This is because 2 mol of $H_2O$ is generated when 1 mol of $CO_2$ is methanized, whereas 1 mol of $H_2O$ is generated when 1 mol of CO is methanized, and thus, the hydrocarbon production system 100 that adopts the methanation reaction of CO can suppress latent heat and sensible heat loss of 1 mol of $H_2O$ as a whole system.

By appropriately adjusting the ratio of $H_2O$ and $CO_2$ introduced into the electrolysis reaction unit 10, the reaction conditions (electrolysis voltage, reaction temperature, or the like) of the electrolysis reaction unit 10, the reaction conditions (amount of catalyst used, GHSV, reaction temperature, or the like) of the reverse water-gas shift reaction unit 20, or the like, the hydrogen/carbon monoxide ($[H_2/CO]$) ratio at the outlet of the reverse water-gas shift reaction unit 20 can be adjusted to a value (for example, $H_2/CO = 3$ which is the equivalent ratio of the methanation reaction of CO, or the like) suitable for the second catalytic reaction unit 30 (hydrocarbon synthesis reaction unit) in the subsequent stage.

[Install Heat Exchanger between Electrolysis Reaction Unit and Reverse Water-Gas Shift Reaction Unit]

In the descriptions so far, the example in which the electrolysis reaction unit 10 and the first catalytic reaction unit (reverse water-gas shift reaction unit) 20 are integrated has been mainly described, however, it is possible to adopt a configuration in which both units 10 and 20 are set as separate units and a heat exchanger 11 is provided between both units 10 and 20 so that heat can be exchanged between both units. This configuration is illustrated in Fig. 6 corresponding to Fig. 1. A hollow double line illustrates the heat transfer between both units. In this configuration, the temperature of each of the units 10 and 20 can be appropriately controlled.

The inventors have called the system including the electrolysis reaction unit 10 and the reverse water-gas shift reaction unit 20 described so far as an "electrolysis reaction system".

[Second Catalytic Reaction Unit (Hydrocarbon Synthesis Reaction Unit)]

At least $H_2$ and CO flow into the second catalytic reaction unit 30 (hydrocarbon synthesis reaction unit), and hydrocarbons (methane and various hydrocarbons having two or more carbon atoms) and the like are generated by the catalytic reaction.

(Example Of Hydrocarbon Synthesis Catalyst)

As an activity test of the catalyst (hydrocarbon synthesis catalyst cat2) used in the second catalytic reaction unit 30, the inventors conducted the following evaluation test 1, evaluation test 2, and evaluation test 3.

As an example of the hydrocarbon synthesis catalyst cat2, a catalyst was prepared by variously changing the carrier and the catalytically active component. As the catalytically active component ca2, those obtained by adding Mo, V, Fe, Co, and the like to Ru and Ru, and Ni were examined. As the carrier cb2, $ZrO_2$, $Al_2O_3$, $SiO_2$, MgO, and $TiO_2$ were examined.

(Catalyst Preparation)

The preparation of the hydrocarbon synthesis catalyst cat2 was also the method adopted as described with reference to Figs. 11 and 12.

That is, a water-soluble ruthenium compound (ruthenium nitrate, ruthenium chloride, ruthenium sulfate, ruthenium ammonium sulfate, ruthenium acetate, ruthenium oxalate, ruthenium citrate, or the like) is quantified and dissolved according to the composition of the target catalyst to obtain an aqueous solution. Further, when molybdenum, vanadium, iron, and cobalt are supported as further catalytically active components, these water-soluble metal compounds are similarly quantified to obtain a dissolved aqueous solution. Using the aqueous solution, for example, by impregnating and supporting the catalytically active component on carrier particles ($ZrO_2$, $Al_2O_3$, $SiO_2$, MgO, $TiO_2$) having a predetermined amount, and carrying out necessary treatment steps such as a drying treatment, a calcination treatment, and a reduction treatment, the hydrocarbon synthesis catalyst cat2 can be obtained.

Using ruthenium chloride aqueous solution, ammonium molybdate aqueous solution, vanadyl oxalate aqueous solution, iron nitrate aqueous solution, and cobalt nitrate aqueous solution, respectively, and when both ruthenium and catalytically active components other than ruthenium are supported, the catalysts of the following examples were prepared using a sequential carrier method (a two-step carrier method in which a catalytically active component other than ruthenium is first supported on a carrier and then ruthenium is supported).

(Evaluation Test 1)

In the evaluation test 1, a mixed gas containing 12.4% CO, 24.8% $CO_2$, 37.2% $H_2$, 12.4% $H_2O$ and the balance being $N_2$ was used as the reaction gas, GHSV was set to 4000/h (WET base), and the activity test of the hydrocarbon synthesis catalyst cat2 was carried out at a reaction temperature of 275°C to 360°C. In this case, the reaction gas is an example obtained by assuming a model in which a co-electrolysis reaction between water and carbon dioxide in the electrolysis reaction unit 10 is carried out under the conditions that an electrolysis reaction rate of carbon dioxide is low, and a mixed gas of CO, $CO_2$, $H_2$, and $H_2O$ after the reverse water-gas shift reaction of carbon dioxide is carried out in the reverse water-gas shift reaction unit 20 installed in the subsequent stage is introduced into the hydrocarbon synthesis reaction unit 30 to carry out the hydrocarbon synthesis reaction.

The following two indicators were adopted when organizing the test results.

1. $CO_2$ removal assumed hydrocarbon conversion rate = [number of carbons in hydrocarbons in outlet gas]/[number of carbons in outlet gas - number of carbons in outlet $CO_2$]

This indicator is an indicator illustrating the conversion rate to hydrocarbons when $CO_2$ is removed from the outlet gas of the hydrocarbon synthesis reaction unit 30 obtained by the catalytic reaction, and it is preferable that this indicator is high.

2. C1-C4 calorific value (MJ/Nm$^3$) = $\Sigma(Nn \times HN)/\Sigma Nn$

Nn [mol]: number of moles of Cn hydrocarbon in gas of catalytic reaction unit (n = 1 to 4)
HN [MJ/m$^3$(N)]: calorific value of Cn hydrocarbon in gas of catalytic reaction unit
[H1=39.8, H2=69.7, H3=99.1, H4=128.5]

This indicator is an indicator illustrating amounts of C1 to C4 components contained in the outlet gas of the hydrocarbon synthesis reaction unit 30 obtained by the catalytic reaction, and when this value exceeds 39.8, it can be confirmed that hydrocarbons such as ethane, propane, and butane are generated in addition to methane.
Regarding the evaluation test 1, Tables 3 and 4 illustrated below illustrate Examples B 1 to B3 of the hydrocarbon synthesis catalyst cat2 in the present invention.

[Table 3]

| | Catalyst | Carrier | Active component supported amount (wt%) | BET surface area (m$^2$/g) | CO adsorption amount (ml/g) |
|---|---|---|---|---|---|
| Example B1 | Ru/Al$_2$O$_3$ | Al$_2$O$_3$ | Ru: 0.4 | 87.4 | 0.66 |
| Example B2 | Ru/Mo/Al$_2$O$_3$ | Al$_2$O$_3$ | Ru: 0.6, Mo: 0.7 | 88.2 | 1.06 |
| Example B3 | Ru/V/Al$_2$O$_3$ | Al$_2$O$_3$ | Ru: 0.7, V: 1.2 | 91.1 | 1.20 |

[Table 4]

| | Catalyst | Indicator | Reaction temperature (°C) | | | |
|---|---|---|---|---|---|---|
| | | | 275 | 310 | 335 | 360 |
| Example B1 | Ru/Al$_2$O$_3$ | CO$_2$ removal assumed hydrocarbon conversion rate (%) | 12.4 | | | 100.0 |
| | | C$_1$-C$_4$ calorific value (MJ/Nm$^3$) | 44.9 | | | 39.8 |
| Example B2 | Ru/Mo/Al$_2$O$_3$ | CO$_2$ removal assumed hydrocarbon conversion rate (%) | | | 99.8 | |
| | | C$_1$-C$_4$ calorific value (MJ/Nm$^3$) | | | 39.9 | |
| Example B3 | Ru/V/Al$_2$O$_3$ | CO$_2$ removal assumed hydrocarbon conversion rate (%) | | 90.0 | | |
| | | C$_1$-C$_4$ calorific value (MJ/Nm$^3$) | | 42.1 | | |

As illustrated in Tables 3 and 4, it was confirmed that hydrocarbons could be synthesized using a catalyst in which ruthenium was supported on an alumina carrier or a catalyst in which molybdenum or vanadium was supported in addition to ruthenium as a hydrocarbon synthesis catalyst cat2 from the mixed gas of CO, $CO_2$, $H_2$, and $H_2O$.
From the above results, it was confirmed that the above-mentioned hydrocarbon production system 100 could generate a high-calorie gas having a C1-C4 calorific value of 39 MJ/Nm$^3$ or more.

(Evaluation Test 2)

In the evaluation test 2, a mixed gas containing 0.45% CO, 18.0% $CO_2$, 71.55% $H_2$, and 10.0% $H_2O$ was used as the reaction gas, GHSV was set to 5000/h (DRY base), and the activity test of the hydrocarbon synthesis catalyst cat2 was carried out at a reaction temperature of about 230°C to about 330°C. In this case, the reaction gas is an example obtained by assuming a model in which the mixed gas obtained when the co-electrolysis reaction of water and carbon dioxide is carried out in the electrolysis reaction unit 10 under the conditions that the electrolysis reaction rate of carbon dioxide is low is introduced into the hydrocarbon synthesis reaction unit 30 to carry out a hydrocarbon synthesis reaction.
The following two indicators were adopted when organizing the test results.

$$1. \text{ Hydrocarbon conversion rate} = [\text{number of carbons in hydrocarbons in outlet gas}]/[\text{number of carbons in outlet gas}]$$

This indicator is an indicator illustrating the ratio of the number of carbons converted into hydrocarbons without being converted into $CO_2$ among the total carbons flowing in, and it is preferable that this indicator is high.

$$2. \ CO_2 \text{ removal assumed hydrocarbon conversion rate} = [\text{number of carbons in hydrocarbons in outlet gas}]/[\text{number of carbons in outlet gas} - \text{number of carbons in outlet } CO_2]$$

This indicator is an indicator illustrating the conversion rate to hydrocarbons when $CO_2$ is removed from the outlet gas of the hydrocarbon synthesis reaction unit obtained by the catalytic reaction, and it is preferable that this indicator is also high.
For the evaluation test 2, the used catalysts (Examples B4 to B16) are illustrated in Table 5, and the test results are illustrated in Table 6.

[Table 5]

| | Catalyst | Carrier | Active component supported amount (wt.%) | BET surface area ($m^2/g$) | CO adsorption amount (ml/g) |
|---|---|---|---|---|---|
| Example B4 | $Ru/Al_2O_3$ | $Al_2O_3$ | Ru: 1.3 | 109.8 | 0.47 |
| Example B5 | $Ru/SiO_2$ | $SiO_2$ | Ru: 1.0 | 212.3 | 0.13 |
| Example B6 | Ru/MgO | MgO | Ru: 1.3 | 24.7 | 0.15 |
| Example B7 | $Ru/TiO_2$ | $TiO_2$ | Ru: 1.2 | 64.7 | 0.71 |
| Example B8 | $Ru/Al_2O_3$ | $Al_2O_3$ | Ru: 2.3 | 114.5 | 0.97 |
| Example B9 | $Ru/Mo/Al_2O_3$ | $Al_2O_3$ | Ru: 1.4, Mo: 1.5 | 131.4 | 0.47 |
| Example B10 | $Ru/V/Al_2O_3$ | $Al_2O_3$ | Ru: 1.2, V: 2.1 | 108.3 | 0.45 |
| Example B11 | $RuMo/Al_2O_3$ | $Al_2O_3$ | Ru: 2.5, Mo: 1.7 | 115.5 | 1.24 |
| Example B12 | $Ru/V/ZrO_2$ | $ZrO_2$ | Ru: 1.1, V: 1.4 | 46.4 | 0.62 |
| Example B13 | $Ru/V/Al_2O_3$ | $Al_2O_3$ | Ru: 1.2, V: 3.9 | 118.0 | 0.63 |

(continued)

|  | Catalyst | Carrier | Active component supported amount (wt.%) | BET surface area $(m^2/g)$ | CO adsorption amount (ml/g) |
|---|---|---|---|---|---|
| Example B14 | $Ru/V/TiO_2$ | $TiO_2$ | Ru: 1.2, V: 1.4 | 57.2 | 1.19 |
| Example B15 | $Ru/Mo/TiO_2$ | $TiO_2$ | Ru: 1.2, Mo: 1.2 | 58.1 | 1.21 |
| Example B16 | $Ni/Al_2O_3$ | $Al_2O_3$ | Ni: 13.0 | 95.7 | 0.01 |

[Table 6]

| | Catalyst | Indicator | Reaction temperature (°C) | | | | | | | | | | | | | | | |
|---|---|---|---|---|---|---|---|---|---|---|---|---|---|---|---|---|---|---|
| | | | 233 | 249 | 257 | 273 | 274 | 276 | 277 | 278 | 287 | 289 | 299 | 302 | 308 | 309 | 317 | 331 |
| Example B4 | $Ru/Al_2O_3$ | Hydrocarbon conversion rate (%) | | | | | | | | | | | 78.6 | | | 80.8 | | 82.0 |
| | | $CO_2$ removal assumed hydrocarbon conversion rate (%) | | | | | | | | | | | 99.9 | | | 99.9 | | 999 |
| Example B5 | $Ru/SiO_2$ | Hydrocarbon conversion rate (%) | | | | | | | | | | | | 3.7 | | | | |
| | | $CO_2$ removal assumed hydrocarbon conversion rate (%) | | | | | | | | | | | | 46.9 | | | | |
| Example B6 | Ru/MgO | Hydrocarbon conversion rate (%) | | | | | | | | 4.2 | | | | | | | | |
| | | $CO_2$ removal assumed hydrocarbon conversion rate (%) | | | | | | | | 78.0 | | | | | | | | |
| Example B7 | $Ru/TiO_2$ | Hydrocarbon conversion rate (%) | | | | | | 64.2 | | | | | | | | | | |
| | | $CO_2$ removal assumed hydrocarbon conversion rate (%) | | | | | | 99.9 | | | | | | | | | | |

EP 4 316 655 A1

19

(continued)

| Catalyst | Indicator | Reaction temperature (°C) | | | | | | | | | | | | | | | |
|---|---|---|---|---|---|---|---|---|---|---|---|---|---|---|---|---|---|
| | | 233 | 249 | 257 | 273 | 274 | 276 | 277 | 278 | 287 | 289 | 299 | 302 | 308 | 309 | 317 | 331 |
| Example B8 Ru/Al$_2$O$_3$ | Hydrocarbon conversion rate (%) | | | | | | | | | 88.4 | | | | | | | |
| | CO$_2$ removal assumed hydrocarbon conversion rate (%) | | | | | | | | | 100.0 | | | | | | | |
| Example B9 Ru/Mo/Al$_2$O$_3$ | Hydrocarbon conversion rate (%) | 14.2 | | | 74.6 | | | | | | | | | | | | |
| | CO$_2$ removal assumed hydrocarbon conversion rate (%) | 99.8 | | | 100.0 | | | | | | | | | | | | |
| Example B10 Ru/V/Al$_2$O$_3$ | Hydrocarbon conversion rate (%) | | | 74.4 | | | | | | | | | | | | | |
| | CO$_2$ removal assumed hydrocarbon conversion rate (%) | | | 100.0 | | | | | | | | | | | | | |
| Example B11 Ru/Mo/Al$_2$O$_3$ | Hydrocarbon conversion rate (%) | | 87.1 | | | | | | | | | | | | | | |
| | CO$_2$ removal assumed hydrocarbon conversion rate (%) | | 100.0 | | | | | | | | | | | | | | |

| | Catalyst | Indicator | Reaction temperature (°C) | | | | | | | | | | | | | | | |
|---|---|---|---|---|---|---|---|---|---|---|---|---|---|---|---|---|---|---|
| | | | 233 | 249 | 257 | 273 | 274 | 276 | 277 | 278 | 287 | 289 | 299 | 302 | 308 | 309 | 317 | 331 |
| Example B12 | Ru/V/ZrO$_2$ | Hydrocarbon conversion rate (%) | | | | | | | 87.8 | | | | | | | | | |
| | | CO$_2$ removal assumed hydrocarbon conversion rate (%) | | | | | | | 100.0 | | | | | | | | | |
| Example B13 | Ru/V/Al$_2$O$_3$ | Hydrocarbon conversion rate (%) | | | | | 78.8 | | | | | | | | | | | |
| | | CO$_2$ removal assumed hydrocarbon conversion rate (%) | | | | | 99.9 | | | | | | | | | | | |
| Example B14 | Ru/V/TiO$_2$ | Hydrocarbon conversion rate (%) | | | | | | | | | | | | | 81.7 | | | |
| | | CO$_2$ removal assumed hydrocarbon conversion rate (%) | | | | | | | | | | | | | 99.9 | | | |
| Example B15 | Ru/Mo/TiO$_2$ | Hydrocarbon conversion rate (%) | | | | | | | | | | 75.2 | | | | | | |
| | | CO$_2$ removal assumed hydrocarbon conversion rate (%) | | | | | | | | | | 99.8 | | | | | | |

EP 4 316 655 A1

(continued)

| | Catalyst | Indicator | Reaction temperature (°C) | | | | | | | | | | | | | | | |
|---|---|---|---|---|---|---|---|---|---|---|---|---|---|---|---|---|---|---|
| | | | 233 | 249 | 257 | 273 | 274 | 276 | 277 | 278 | 287 | 289 | 299 | 302 | 308 | 309 | 317 | 331 |
| Example B16 | $Ni/Al_2O_3$ | Hydrocarbon conversion rate (%) | | | | | | | | | | | | | | | 26.7 | |
| | | $CO_2$ removal assumed hydrocarbon conversion rate (%) | | | | | | | | | | | | | | | 96.2 | |

(Evaluation Test 3)

In the evaluation test 3, a mixed gas ($H_2$/CO = 3) containing $H_2$ and CO in a ratio of 3:1 (volume ratio) was used as the reaction gas, the GHSV was set to 2000/h, and the activity test of the hydrocarbon synthesis catalyst cat2 was carried out at the reaction temperature of 235°C to about 330°C. In this activity test, a catalyst (Examples B17 and B18) in which iron or cobalt was supported on a titania carrier in addition to ruthenium was used. In this case, the reaction gas is an example obtained by assuming a model in which a mixed gas obtained by adding carbon monoxide to hydrogen obtained by electrolyzing water in the electrolysis reaction unit 10, and a mixed gas of hydrogen and carbon monoxide obtained by separating water and carbon dioxide as needed from the gas obtained by carrying out a co-electrolysis reaction of water and carbon dioxide are introduced into the hydrocarbon synthesis reaction unit 30 to carry out the hydrocarbon synthesis reaction.

The results of the evaluation test 3 are illustrated in Table 7.

[Table 7]

| | Catalyst | Indicator | Reaction temperature (°C) | | | |
|---|---|---|---|---|---|---|
| | | | 235 | 250 | 278 | 327 |
| Example B17 | 2 wt.%Ru/2 wt. %Fe/TiO$_2$ | $CO_2$ removal assumed hydrocarbon conversion rate (%) | 11.6 | | 99.9 | 99.9 |
| | | $C_1$-$C_4$ calorific value (MJ/Nm$^3$) | 59.0 | | 47.1 | 42.16 |
| Example B18 | 2 wt.%Ru/2 wt. %Co/TiO$_2$ | $CO_2$ removal assumed hydrocarbon conversion rate (%) | | 99.0 | | |
| | | $C_1$-$C_4$ calorific value (MJ/Nm$^3$) | | 46 | | |

As illustrated in Table 7, it was confirmed that hydrocarbons can be synthesized from a mixed gas containing $H_2$ and CO using a catalyst in which ruthenium and iron or cobalt are supported on a titania carrier as a hydrocarbon synthesis catalyst cat2.

It was confirmed that the above-mentioned hydrocarbon production system 100 can generate a high-calorie gas having a C1-C4 calorific value of 39 MJ/Nm$^3$ or more.

From the above results, as illustrated above, a catalyst in which at least ruthenium is supported as the catalytically active component ca2 on the metal oxide carrier cb2 can be used in the second catalytic reaction unit 30 (hydrocarbon synthesis reaction unit). Further, it is preferable to support at least one of molybdenum, vanadium, iron, and cobalt as the catalytically active component ca2.

It was found that, preferably, the hydrocarbon synthesis catalyst cat2 was a catalyst in which at least ruthenium was supported on the metal oxide carrier cb2, the supported amount of ruthenium was 0.1% by weight or more and 5% by weight or less, and at least one of molybdenum, vanadium, iron, and cobalt as the catalytically active component ca2 was supported on the metal oxide carrier cb2 in addition to ruthenium.

Here, the supported amount of at least one of the molybdenum, vanadium, iron, and cobalt can be 0.2% by weight or more and 6% by weight or less.

Further, in hydrocarbon synthesis catalysts cat2, the adsorption amount of carbon monoxide of the highly active catalyst was 0.4 ml/g or more.

[Heavy Hydrocarbon Separation Unit]

When the gas reaching the heavy hydrocarbon separation unit 35 is cooled, the heavy hydrocarbons contained in the gas released from the hydrocarbon synthesis reaction unit 30 are condensed and the heavy hydrocarbons can be taken out to the outside. For example, in the hydrocarbon synthesis reaction unit 30 using the 2 wt.% Ru/2 wt.% Fe/TiO$_2$ catalyst illustrated in Example B17 above, when a mixed gas ($H_2$/CO = 3) containing $H_2$ and CO in a ratio of 3:1 (volume ratio) was introduced and the reaction was carried out at 275°C, a linear higher aliphatic hydrocarbon having an average chain length of 26 carbon atoms could be extracted from the heavy hydrocarbon separation unit 35. Moreover, when the reaction was carried out at 325°C, a linear higher aliphatic hydrocarbon having an average chain length of 18 carbon atoms could be extracted from the heavy hydrocarbon separation unit 35.

[Water Separation Unit]

A condenser is arranged in the water separation unit 40, and the gas containing $H_2O$ flowing in is adjusted to a predetermined temperature and pressure to be condensed and water is taken out to the outside.

[Carbon Dioxide Separation Unit]

For example, PSA is arranged in this unit 50, and the gas containing $CO_2$ flowing in is adsorbed to the adsorbent under a predetermined temperature and pressure to separate $CO_2$, the separated $CO_2$ is separated from the adsorbent, and thus, $CO_2$ is favorably separated. The separated $CO_2$ can be returned to the front of the electrolysis reaction unit 10 and reused via the carbon dioxide return path 51.

It is also possible to use PSA or the like to make the carbon dioxide separation unit and the water separation unit the same separation unit.

[Another Embodiment]

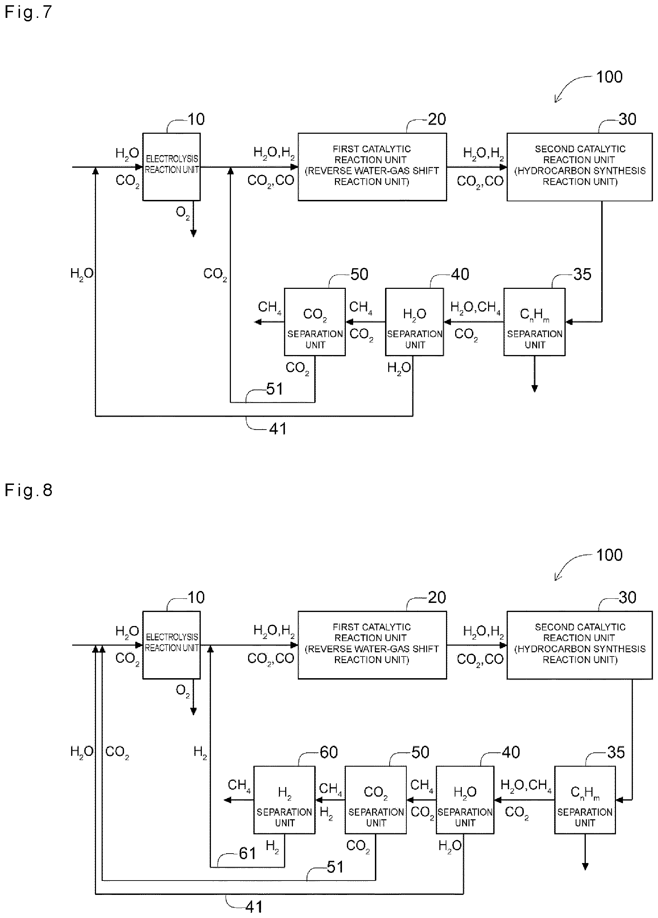

(1) In the above embodiment, $CO_2$ separated in the carbon dioxide separation unit 50 is returned to the front of the electrolysis reaction unit 10. However, in the hydrocarbon production system 100 according to the present invention, since the conversion of $CO_2$ to CO is mainly performed by the reverse water-gas shift reaction unit 20, a return destination of $CO_2$ may be in front of the reverse water-gas shift reaction unit 20. This configuration is illustrated in Fig. 7.

(2) In the above embodiment, $H_2$ in the gas obtained from the hydrocarbon synthesis reaction unit 30 is not particularly described. However, a hydrogen separation unit (described as $H_2$ separation unit in the drawing) 60 that separates $H_2$ using a hydrogen separation membrane or the like may be provided to separate $H_2$ and use $H_2$ separately. This configuration is illustrated in Fig. 8. In this example, the return destination of $H_2$ separated by the hydrogen separation unit 60 may be provided in front of the reverse water-gas shift reaction unit 20 so that $H_2$ is used for the reverse water-gas shift reaction.

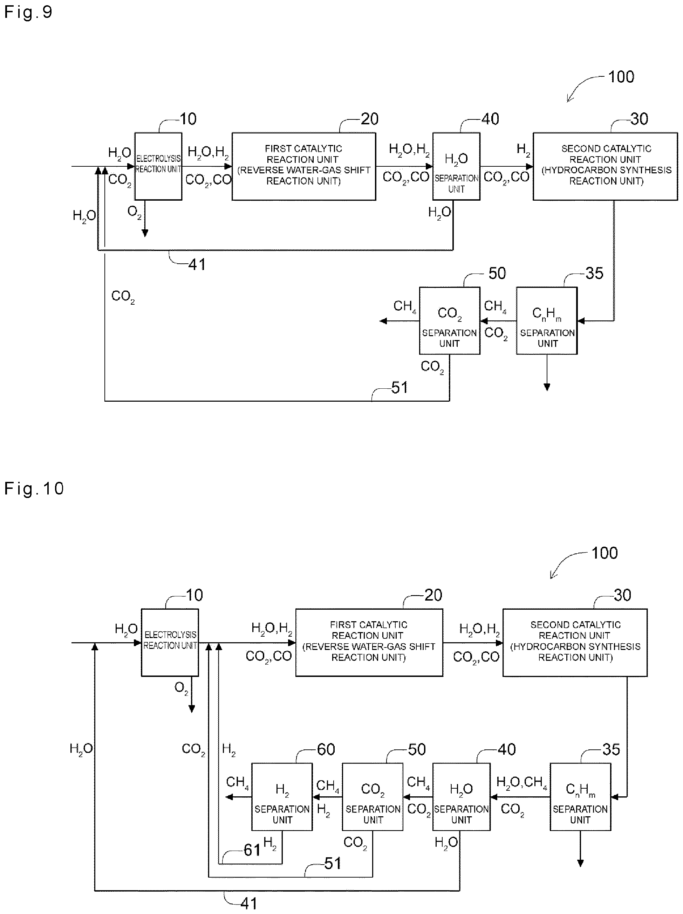

(3) In the above embodiment, the water separation unit 40 is provided on the lower side of the hydrocarbon synthesis reaction unit 30. However, as illustrated in Fig. 9, the water separation unit 40 may be provided between the reverse water-gas shift reaction unit 20 and the hydrocarbon synthesis reaction unit 30. The main function of the water separation unit 40 is to facilitate the hydrocarbon synthesis reaction.

(4) In the above embodiment, an example in which both $H_2O$ and $CO_2$ are supplied to the electrolysis reaction unit 10 and subjected to the electrolysis reaction is illustrated. However, as illustrated in Fig. 10, a system may be used in which only $H_2O$ is supplied to the electrolysis reaction unit 10 to be subjected to the electrolysis reaction. In this case, the carbon consumed in the hydrocarbon synthesis is input to the reverse water-gas shift reaction unit 20 as carbon dioxide.

(5) In the above embodiment, an example in which a solid oxide type electrolysis cell is used as the electrolysis cell 1 in the electrolysis reaction unit 10 is illustrated. However, as the electrolysis cell 1, an alkaline type electrolysis cell, a polymer film type electrolysis cell, or the like may be used.

(6) In the above embodiment, the electrolysis reaction unit 10 and the first catalytic reaction unit 20 are integrated. However, in addition to the reaction units 10.20, the second catalytic reaction unit 30 may be integrated. A configuration example in this case is illustrated in Fig. 13. Incidentally, in Fig. 13, a reference numeral 30a indicates a coating layer of the hydrocarbon synthesis catalyst cat2.

Also, in the case of this configuration, each of the reaction units 10, 20, and 30 can be configured on the metal support 4 and the supply path forming member 5, and the metal support 4 is supposed to act as a separator for separating the generated hydrocarbon and oxygen.

(7) In the above embodiment, an example of synthesizing a hydrocarbon such as methane in the hydrocarbon synthesis reaction unit 30 is illustrated. However, depending on how the hydrocarbon synthesis catalyst used in the hydrocarbon synthesis reaction unit 30 is selected, it is also possible to synthesize a chemical raw material from hydrogen and carbon monoxide introduced into the hydrocarbon synthesis reaction unit 30.

(8) In the above embodiment, the precursor obtained by impregnating the carrier cb1 containing alumina as a main component with nickel as the catalytically active component ca1 to be supported on the carrier cb1 is described. However, it is possible to obtain a precursor by adding at least a nickel component to alumina using a method other than the impregnation-supporting method, such as a coprecipitation method and a kneading method.

[Industrial Applicability]

The present invention provides a reverse water-gas shift catalyst that can be used at high temperatures. This reverse water-gas shift catalyst can be suitably used in an electrolysis reaction system and a hydrocarbon production system.

Reference Signs List

1: Electrolysis cell
1a: Electrolyte layer
2: Electrode layer
3: Counter electrode layer
4: Metal support (support/separator)
4a Hole (through hole)
5: Supply path forming member (separator)
6: Supply path forming member (separator)
10: Electrolysis reaction unit
20: First catalytic reaction unit (reverse water-gas shift reaction unit)
20a: Coating layer
30: Second catalytic reaction unit (hydrocarbon synthesis reaction unit)
40: Water separation unit
50: Carbon dioxide separation unit
60: Hydrogen separation unit
U: Electrolysis cell unit
cat1: Reverse water-gas shift catalyst
ca1: Catalytically active component (metal component)
cb1: Carrier (metal oxide)
cat2: Hydrocarbon synthesis catalyst
ca2: Catalytically active component (metal component)
cb2: Carrier (metal oxide)

**Claims**

1. A method for producing a reverse water-gas shift catalyst, the method comprising calcinating a precursor obtained by adding at least a nickel component to alumina at a temperature of 500°C or higher.

2. A method for producing a reverse water-gas shift catalyst, the method comprising:
executing an impregnation-supporting step of impregnating a carrier containing alumina as a main component with nickel to be supported on the carrier and simultaneously calcinating a precursor obtained in the impregnation-supporting step at a temperature of 500°C or higher.

3. A reverse water-gas shift catalyst comprising a $NiAl_2O_4$ phase as a crystalline phase.

4. A reverse water-gas shift catalyst comprising an aluminum element and a nickel element, which have a CO adsorption amount of 0.45 mL or less per 1 g of the catalyst.

5. A reverse water-gas shift catalyst comprising an aluminum element and a nickel element,
wherein the nickel has an average crystal particle size of 10 nm or more.

6. A reverse water-gas shift catalyst comprising an aluminum element and a nickel element, which have a BET surface area of 105 $m^2$ or less.

7. The reverse water-gas shift catalyst according to any one of claims 4 to 6, further comprising a $NiAl_2O_4$ phase as a crystalline phase.

8. An electrolysis reaction system comprising at least:

   a reverse water-gas shift reaction unit including at least the reverse water-gas shift catalyst according to any one of claims 3 to 6; and
   an electrolysis reaction unit.

9. The electrolysis reaction system according to claim 8,
   wherein the electrolysis reaction unit has an electrolysis cell unit in which at least an electrode layer, an electrolyte layer, and a counter electrode layer are formed on a support.

10. The electrolysis reaction system according to claim 9,
    wherein the support is a metal.

11. A hydrocarbon production system for producing hydrocarbon from water and carbon dioxide, the hydrocarbon production system comprising at least:

    a reverse water-gas shift reaction unit including at least the reverse water-gas shift catalyst according to any one of claims 3 to 6;
    an electrolysis reaction unit; and
    a hydrocarbon synthesis reaction unit.

12. A method of using a reverse water-gas shift catalyst, the method comprising subjecting the reverse water-gas shift catalyst according to any one of claims 3 to 6 to a reaction after performing a reduction pretreatment.

Fig.1

Fig.2

Fig.3

Fig.4

Fig.5

Fig.6

Fig.7

Fig.8

Fig.9

Fig.10

Fig.11

(a) | IMPREGNATION-SUPPORTING STEP |

cb1,cb2

ca1,ca2

(b) | DRYING/CRUSHING/MOLDING STEP |

ca1,ca2

cb1,cb2

(c) | CALCINATION STEP |

ca1,ca2

cb1,cb2

cat1,cat2

Fig.12

(a) | COATING/CALCINATION STEP

(b) | REDUCTION PRETREATMENT STEP

Fig.13

Fig.14

Fig.15

Fig.16

# INTERNATIONAL SEARCH REPORT

| International application No. |
|---|
| **PCT/JP2022/015817** |

**A. CLASSIFICATION OF SUBJECT MATTER**

*B01J 23/755*(2006.01)i; *B01J 35/10*(2006.01)i; *B01J 37/08*(2006.01)i; *C01B 3/02*(2006.01)i; *C01B 32/40*(2017.01)i; *C07B 61/00*(2006.01)i; *C07C 1/04*(2006.01)i; *C07C 9/04*(2006.01)i; *C07C 9/06*(2006.01)i; *C07C 9/08*(2006.01)i; *C07C 9/10*(2006.01)i; *C25B 1/042*(2021.01)i; *C25B 1/23*(2021.01)i; *C25B 9/23*(2021.01)i; *B01J 23/46*(2006.01)n; *B01J 23/648*(2006.01)n; *B01J 23/652*(2006.01)n; *C25B 11/052*(2021.01)n; *C25B 11/054*(2021.01)n; *C25B 11/067*(2021.01)n; *C25B 11/075*(2021.01)n

FI: B01J23/755 M; B01J35/10 301J; B01J37/08; C01B3/02 Z; C01B32/40; C07B61/00 300; C07C1/04; C07C9/04; C07C9/06; C07C9/08; C07C9/10; C25B1/042; C25B1/23; C25B9/23; B01J23/46 301Z; B01J23/648 Z; B01J23/652 Z; C25B11/052; C25B11/054; C25B11/075; C25B11/067

According to International Patent Classification (IPC) or to both national classification and IPC

**B. FIELDS SEARCHED**

Minimum documentation searched (classification system followed by classification symbols)

B01J21/00-38/74; C01B3/00-6/34; C01B32/40; C07B61/00; C07C1/02-1/12; C07C9/00-9/22; C25B1/00-15/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | YANG, L. et al. Applied Catalysis B: Environmental. 27 March 2018, vol. 232, pp. 464-471, <DOI:10.1016/j.apcatb.2018.03.091> abstract, p. 464, left column, first paragraph to p. 466, left column, fifth paragraph, p. 469, left column, first paragraph to p. 471, left column, third paragraph | 1-7, 12 |
| Y | | 8-11 |

| ✓ | Further documents are listed in the continuation of Box C. | ✓ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **08 June 2022** | **21 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

<div align="center">

**INTERNATIONAL SEARCH REPORT**

</div>

| International application No. |
| --- |
| **PCT/JP2022/015817** |

### C. DOCUMENTS CONSIDERED TO BE RELEVANT

| Category* | Citation of document, with indication, where appropriate, of the relevant passages | Relevant to claim No. |
| --- | --- | --- |
| X | UNDE, R. B. Kinetics and Reaction Engineering Aspects of Syngas Production by the Heterogeneously Catalysed Reverse Water Gas Shift Reaction. Doctoral thesis. University of Bayreuth. Faculty of Engineering Science. [online], 2012, [retrieved on 02 June 2022] <URL:https://epub.uni-bayreuth.de/239/>  p. 20, second paragraph to p. 27, third paragraph, p. 39, fist paragraph to p. 47, second paragraph, p. 55, second paragraph to p. 78, first paragraph, p. 123, first paragraph to p. 124, second paragraph, table 4-1, fig. 2-8, 5-2(B) | 5-6, 8-12 |
| Y | | 8-11 |
| X | JP 07-068171 A (COSMO SOGO KENKYUSHO KK) 14 March 1995 (1995-03-14)  example 1, claims, paragraphs [0001]-[0018] | 1-7, 12 |
| Y | | 8-11 |
| X | CHEN, C. S. et al. Journal of Physical Chemistry A. 08 May 2009, vol. 114, pp. 3773-3781, <DOI:10.1021/jp904434e>  abstract, p. 3773, left column, first paragraph to p. 3774, left column, third paragraph, p. 3775, right column, second paragraph to p. 3777, left column, first paragraph, p. 3781, left column, second paragraph, fig. 1, table 2 | 3-7, 12 |
| Y | | 8-11 |
| X | CHOI, S. et al. Scientific Reports. 25 January 2017, vol. 7, 41207, <DOI:10.1038/srep41207>  abstract, p. 2, second paragraph to p. 4, fourth paragraph, p. 6, third paragraph to p. 7, fourth paragraph, fig. 1-4, supplementary information, table S1 | 6 |
| Y | | 8-11 |
| X | JP 2020-179347 A (TOYOTA CENTRAL RES & DEV) 05 November 2020 (2020-11-05)  preparation example 2, claims, paragraphs [0001]-[0015], [0051] | 1-7, 12 |
| X | AMIR, E. et al. Iranian Journal of Hydrogen & Fuel Cell. 2017, vol. 4, pp. 315-322 , <DOI:10.22104/ijhfc.2017.488>  abstract, p. 316, left column, third paragraph to right column, second paragraph, p. 317, left column, third paragraph, p. 319, right column, second paragraph to p. 321, left column, first paragraph, table 2, fig. 1, 4 | 1-3, 6-7 |
| A | JP 2006-052120 A (REI, Minho) 23 February 2006 (2006-02-23)  table 3 | 1-12 |
| P, Y | WO 2021/201191 A1 (OSAKA GAS CO LTD) 07 October 2021 (2021-10-07)  claims, paragraphs [0006]-[0051], fig. 1-4, 6-10, 13 | 8-11 |

Form PCT/ISA/210 (second sheet) (January 2015)

INTERNATIONAL SEARCH REPORT
Information on patent family members

International application No.

**PCT/JP2022/015817**

| Patent document cited in search report | | | Publication date (day/month/year) | Patent family member(s) | | | Publication date (day/month/year) |
|---|---|---|---|---|---|---|---|
| JP | 07-068171 | A | 14 March 1995 | US example 1, claims, column 1, second paragraph to column 2, 10th paragraph | 5911964 | A | |
| | | | | GB | 2279583 | A | |
| | | | | DE | 4422227 | A | |
| | | | | CA | 2126502 | A | |
| JP | 2020-179347 | A | 05 November 2020 | (Family: none) | | | |
| JP | 2006-052120 | A | 23 February 2006 | US table 3 | 2005/0158236 | A1 | |
| | | | | TW | 200524824 | A | |
| | | | | CN | 1644485 | A | |
| WO | 2021/201191 | A1 | 07 October 2021 | (Family: none) | | | |

Form PCT/ISA/210 (patent family annex) (January 2015)

**EP 4 316 655 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019035102 A